# EUROPÄISCHE PATENTANMELDUNG

(11) **EP 2 266 421 A1**
(43) Veröffentlichungstag der Anmeldung: **29.12.2010**
(21) Anmeldenummer: 10164775.8
(22) Anmeldetag: 02.06.2010
(51) Int. Cl.: A23L 1/226, A61K 8/46, A61Q 13/00, C11B 9/00, C11D 3/50, C07C 321/10

(54) **Verwendung bestimmter Isopropylmethylcyclohexenthiole als Riech- und/oder Aromastoffe**

(30) Priorität: 03.06.2009 DE 102009026698
(71) Anmelder: Symrise AG, 37603 Holzminden (DE)
(72) Erfinder: Ott, Frank, 37603, Holzminden (DE); Kindel, Günter, 37671, Höxter (DE)
(74) Vertreter: Eisenführ, Speiser & Partner

(57) **Zusammenfassung**

Beschrieben werden neue Verbindungen der Formel (A) sowie insbesondere die Verwendung einer Verbindung der Formel (A) oder einer Mischung aus zwei oder mehr Verbindungen der Formel (A), wobei die Thiolgruppe jeder Verbindung der Formel (A) jeweils unabhängig voneinander mit dem Kohlenstoffatom in Position 3, 4, 5 oder 6 verknüpft ist, als Riech- und/oder Aromastoff zum Vermitteln, Modifizieren und/oder Verstärken einer, zwei oder sämtlicher der Geruchs- und/oder Geschmacksnoten Grapefruit, tropische Früchte und schwarze Johannisbeere

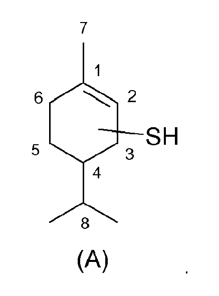

Beschrieben werden ferner Riech- und Aromastoffkompositionen, Zubereitungen und Verfahren.

## Beschreibung

Die vorliegende Erfindung betrifft primär die Verwendung bestimmter Isopropylmethylcyclohexenthiole, nämlich Verbindungen der Formel (A) wie unten abgebildet, als Riech- oder/oder Aromastoffe, insbesondere zum Vermitteln, Modifizieren und/oder Verstärken einer blumigen und/oder fruchtigen Geschmacks- und/oder Geruchsnote, vorzugsweise einer, zwei oder sämtlicher der Geruchs- und/oder Geschmacksnoten Grapefruit, tropische Früchte und Schwarze Johannisbeere.

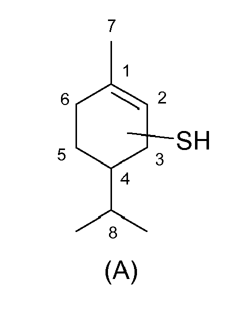

Bevorzugt zu verwendende Verbindungen der Formel (A) werden weiter untern beschrieben.

Zudem betrifft die vorliegende Erfindung die neuen Verbindungen der Formeln (III) und (IV) (wie jeweils weiter unten beschrieben) sowie Mischungen umfassend oder bestehend aus einer oder mehreren Verbindungen der Formel (III) oder (IV) sowie gegebenenfalls einer oder mehrerer Verbindungen der Formel (I) oder (II).

Ein weiterer Aspekt der vorliegenden Erfindung betrifft Riech- oder Aromastoffkompositionen, die eine oder mehrere erfindungsgemäße bzw. erfindungsgemäß zu verwendende Verbindungen der Formel (A) umfassen. Des Weiteren betrifft die vorliegende Erfindung der Ernährung, dem Genuss oder der Mundpflege dienende Zubereitungen und parfümierte Artikel, umfassend eine oder mehrere Verbindungen der Formel (A) oder eine erfindungsgemäße Riech- oder Aromastoffkomposition.

Zudem betrifft die vorliegende Erfindung ein Verfahren zum Vermitteln, Modifizieren und/oder Verstärken eines Geruchs und/oder Geschmacks sowie ein Verfahren zur Herstellung von Verbindungen der Formel (A).

Weitere Aspekte der vorliegenden Erfindung sowie besonders bevorzugte Ausgestaltungen ergeben sich aus der folgenden Beschreibung, den Beispielen sowie den beigefügten Patentansprüchen.

Trotz einer Vielzahl bereits vorhandener Riech- und Aromastoffe besteht in der Parfümindustrie sowie in der Lebensmittel verarbeitenden Industrie auch weiterhin ein genereller Bedarf an neuen Riech- und Aromastoffen, die über ihre primären, nämlich geruchlichen bzw. geschmacklichen Eigenschaften hinaus zusätzliche positive Sekundäreigenschaften besitzen, wie z.B. eine höhere Stabilität unter bestimmten Anwendungsbedingungen, eine höhere Ausgiebigkeit, ein besseres Haftungsvermögen oder aber auch bessere dermatologische und toxikologische Eigenschaften gegenüber vergleichbaren Riech- bzw. Aromastoffen.

Insbesondere besteht ein Bedürfnis an Riech- und Aromastoffen, die geschmackliche bzw. geruchliche Charakteristiken von Citrusfrüchten aufweisen.

Die geschmacklichen Charakteristiken von Citrusfrüchten (*Citrus aurantium*), insbesondere Zitrone, Orange, Apfelsine, Mandarine, Clementine, Grapefruit, Pampelmuse, Limone, Limette, Kumquat, Tangor und Tangelo, werden durch eine Reihe von Mono-, Sesqui-, Di- und Triterpenen geprägt. Eine große Anzahl dieser Verbindungen findet man in den kommerziell erhältlichen Ölen und Extrakten der verschiedenen Früchte wieder.

Eine der prominentesten Verbindungen unter den schwefelhaltigen Terpenen ist das 2-(4-Methyl-cyclohex-3-enyl)-propan-2-thiol, besser bekannt unter dem in der Terpenchemie gebräuchlichen Namen p-Menth-1-en-8-thiol oder auch p-Menthenthiol-1,8 (Verbindung G, siehe unten) (vgl. Helv. Chim. Acta 1982, 65(6), 1785-1794). p-Menthenthiol-1,8 ist im unteren ppb-Bereich sensorisch aktiv und eine wichtige Verbindung für eine typische, frische Grapefruitnote. Es ist jedoch bekannt, dass p-Menthenthiol-1,8 in saurem Medium, wie es auch in der natürlichen Frucht der Fall vorliegt, leicht zum 1,8-Epithio-p-menthan und/oder zum 2,8-Epithio-p-menthan reagiert (vgl. Helv. Chim. Acta 1982, 65(6), 1785-1794), wie im folgenden Schema dargestellt:

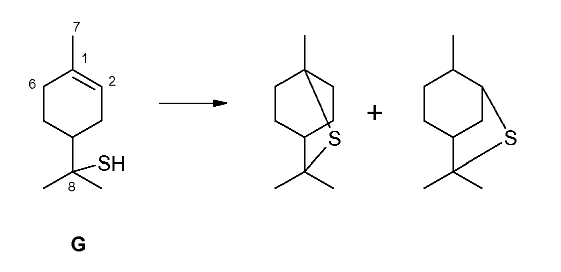

Die dabei gebildeten Episulfide besitzen sensorische Eigenschaften, die gegenüber der Verbindung **G** (häufig) nicht gewünschte Noten wie Lauch, Zwiebel und Durian aufweisen. Daher geht das sensorische Profil von p-Menthenthiol-1,8 (Verbindung **G**), das heißt die frische Grapefruitnote, in kurzer Zeit verloren.

Aufgabe der vorliegenden Erfindung war es daher, neue Riech- oder Aromastoffe zu finden, insbesondere Riech- oder Aromastoffe, die ähnliche sensorische Eigenschaften wie p-Menthenthiol-1,8 aufweisen. Vorzugsweise sollten demnach Riech- oder Aromastoffe gefunden werden, die eine fruchtige Geruchs- und/oder Geschmacksnote, insbesondere eine Grapefruitnote, vermitteln, modifizieren und/oder verstärken können. Weiter bevorzugt sollten dabei solche Aroma- oder Riechstoffe gefunden werden, die eine gegenüber p-Menthenthiol-1,8 höhere Stabilität (in saurem Medium) besitzen.

Weitere der vorliegenden Erfindung zugrundeliegende Aufgaben ergeben sich aus der folgenden Beschreibung, den Beispielen sowie insbesondere den beigefügten Patentansprüchen.

Die primäre Aufgabe der vorliegenden Erfindung wird erfindungsgemäß gelöst durch die Verwendung
- einer Verbindung der Formel (A) 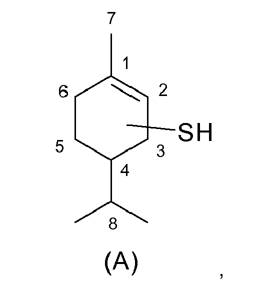 wobei die Thiolgruppe der Verbindung der Formel (A) mit dem Kohlenstoffatom in Position 3, 4, 5 oder 6 (gemäß der gezeigten Nummerierung) verknüpft ist, oder
- einer Mischung aus zwei oder mehr Verbindungen der Formel (A), wobei die Thiolgruppe jeder Verbindung der Formel (A) jeweils unabhängig voneinander mit dem Kohlenstoffatom in Position 3, 4, 5 oder 6 (gemäß der gezeigten Nummerierung) verknüpft ist,
   als Riech- und/oder Aromastoff zum Vermitteln, Modifizieren und/oder Verstärken einer blumigen und/oder fruchtigen Geruchs- und/oder Geschmacksnoten, vorzugsweise zum Vermitteln, Modifizieren und/oder Verstärken einer, zwei oder sämtlicher der Geruchs- und/oder Geschmacksnoten Grapefruit, tropische Früchte und schwarze Johannisbeere.

In der vorstehend gezeigten Darstellung der Formel (A) entsprechen die Ziffern 1 bis 8 einer willkürlichen, nicht notwendigerweise IUPAC-konformen Nummerierung der Kohlenstoffatome.

Bei den Verbindungen der Formel (A) (wie oben gezeigt) handelt es sich je nach Position der Thiolgruppe um die Verbindungen der Formeln (I) bis (IV):

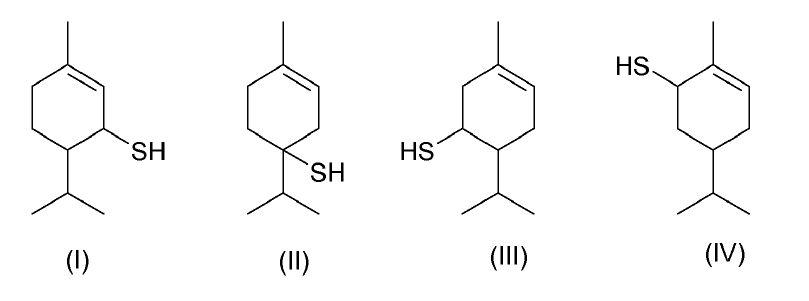

Die erfindungsgemäß zu verwendenden Verbindungen der Formel (A) umfassen demnach die Verbindungen
- 4-lsopropyl-1-methyl-cyclohex-1-en-3-thiol (Verbindung der Formel (I), auch p-Menth-1-en-3-thiol oder p-Menthenthiol-1,3),
- 4-lsopropyl-1-methyl-cyclohex-1-en-4-thiol (Verbindung der Formel (II), auch p-Menth-1-en-4-thiol oder p-Menthenthiol-1,4),
- 4-lsopropyl-1-methyl-cyclohex-1-en-5-thiol (Verbindung der Formel (III), auch p-Menth-1-en-5-thiol oder p-Menthenthiol-1,5) und
- 4-lsopropyl-1-methyl-cyclohex-1-en-6-thiol (Verbindung der Formel (IV), auch p-Menth-1-en-6-thiol oder p-Menthenthiol-1,6)
   sowie deren Enantiomere und Diastereomere.

In Flavour and Fragrance Journal 1993, 8, 289-294 wurde die Umsetzung von einigen Terpenen mit Schwefelwasserstoff und Katalysatoren wie Aluminiumtrichlorid untersucht. Dabei wurden schwefelhaltige Terpene erzeugt und aus dem Rohprodukt per Massenspektrometrie analysiert, wobei auch Verbindungen der Formel (I) (wie oben gezeigt) in unterschiedlichen Mengenanteilen in den dort hergestellten Produktgemischen gefunden wurden. Je nach Wahl der eingesetzten Terpene wurden dort Reaktionsmischungen erhalten, die neben einer oder mehrerer Verbindungen der Formel (I) zusätzlich einen oder mehrere Riech- oder Aromastoffe enthielten wie Limonen, alpha-Pinen, alpha-Terpinen, gamma-Terpinen, 3-Caren, p-Cymen, 1,8-Cineol (Eucalyptol) sowie p-Menthenthiol-1,8 (Verbindung G, vgl. oben). Zudem werden in der genannten Veröffentlichung eine Verbindung der Formel (II) (wie oben gezeigt) und Reaktionsmischungen enthaltend eine solche Verbindung beschrieben. Die Autoren weisen in dieser Veröffentlichung ausdrücklich darauf hin, dass die Untersuchung der sensorischen Eigenschaften der erhaltenen Verbindungen nicht Ziel ihrer Arbeiten war, so dass sich in dieser Literaturstelle auch keine Beschreibung der sensorischen Eigenschaften von Verbindungen der Formeln (I) oder (II) finden.

Die durch die Umsetzung ausgehend jeweils von Limonen, alpha-Pinen, alpha-Terpinen, gamma-Terpinen bzw. 3-Caren mit Schwefelwasserstoff in Gegenwart von Aluminiumtrichlorid gemäß Flavour and Fragrance Journal 1993, 8, 289-294 hergestellten bzw. herstellbaren Reaktionsmischungen sowie sonstige in diesem Dokument offenbarte Mischungen (z.B. die für die Flüssigkeitschromatographie eingesetzten Mischungen mit Hexan oder Ethylacetat) sind in der vorliegenden Erfindung vom Schutz ausdrücklich ausgenommen. Diese Mischungen sind zudem keine (erfindungsgemäßen) der Ernährung, dem Genuss oder der Mundpflege dienende Zubereitungen oder parfümierte Artikel (wie weiter unten beschrieben).

In nachfolgender Tabelle sind die in eigenen Untersuchungen ermittelten sensorischen Eigenschaften der erfindungsgemäßen Verbindungen der Formeln (I) bis (IV) angegeben. Für interne Verkostungen zur Ermittlung der sensorischen Eigenschaften wurden die zu verkostenden Verbindungen der Formeln (I) bis (IV) jeweils in einer Menge von 500 ppb in 5%iger Zuckerlösung und in 0,5%iger Kochsalzlösung eingesetzt. Die Stärke der jeweiligen (sensorischen) Eindrücke ist gemäß einer Skala von 1 (sehr schwach) bis 9 (sehr stark) angegeben.

| **Verbindung** | **Sensorische Beschreibung** |
|---|---|
| **Formel (I)** | Impact (7), Intensität (6), Fülle (6), haftfest (5), Grapefruit (5), Bucco (5), schwefelig (5), überreif (5), schalig (5), tropische Früchte (4), Passions-frucht (4), geröstet (4), fruchtig (3), schwarze Johannisbeere (3), Citrus (3), schwefelig (3) |
| **Formel (II)** | Intensität (6), Impact (6), haftfest (6), Fülle (5), Grapefruit (5), schalig (5), schwefelig (4), Bucco (3), tropische Früchte (3), schwarze Johannisbeere (3), Citrus, Limette (2) |
| **Formel (III)** | haftfest (7), Intensität (8), Impact (7), Fülle (6), Citrus (6), Grapefruit (6), Bucco (6), tropische Früchte (5), krautig (5), fruchtig (5), Passionsfrucht (4), schwarze Johannisbeere (4), süß (3), schwefelig (3) |
| **Formel (IV)** | Intensität (8), Impact (8), Grapefruit (8), schwefelig (8), haftfest (7), Fülle (7), schwarze Johannisbeere (7), tropische Früchte (7), Bucco (6), Guave (6), Cassis (6), Sesam, geröstet (4), Citrus (2) |

Vergleichend hierzu können die sensorischen Eigenschaften der aus dem Stand der Technik bekannten Verbindung **G** (p-Menthenthiol-1,8, vgl. oben) nach eigenen Untersuchungen wie folgt beschrieben werden:
Grapefruit (8), Intensität (7), Impact (6), haftfest (5), Fülle (5), fruchtig (5), Limette (4), tropische Früchte (4), saftig (3), Citrus (3), Mango (3), schwarze Johannisbeere (3), Bucco (3), Passionsfrucht (3), süß (3), schwefelig (3), Durian (2).

In den eigenen Untersuchungen wurden zudem die sensorischen Eigenschaften der aus Limonen hergestellten, aus dem Stand der Technik bekannten Verbindung L (siehe unten) untersucht

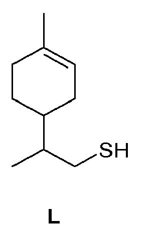

Die Verbindung L zeigte dabei stark fruchtige (in Richtung Apfel und tropische Früchte), schwefelige und krautige Noten, aber auch frisch-grüne und brandig-holzige Aspekte. Das sensorische Profil der Verbindung **L** unterscheidet sich demnach überraschenderweise deutlich von dem sensorischen Profil der erfindungsgemäß zu verwendenden Verbindungen der Formel (A).

Besonders überraschend wurde im Rahmen der eigenen Untersuchungen herausgefunden, dass bei den erfindungsgemäß zu verwendenden Verbindungen der Formel (A) - im Gegensatz zu p-Menthenthiol-1,8 (Verbindung **G,** vgl. oben) - die Grapefruitnote (insbesondere in saurem Medium) stabil erhalten bleibt. Ausgehend von Helv. Chim. Acta 1982, 65(6), 1785-1794 (vgl. oben) war nicht vorherzusehen, dass durch eine im Vergleich zu p-Menthenthiol-1,8 andere Anordnung der Thiolgruppe stabilere Riech- bzw. Aromastoffe erhalten werden.

Zudem wurde im Rahmen eigener Untersuchungen herausgefunden, dass je nach Position der Thiolgruppe, d.h. abhängig davon, ob die Thiolgruppe mit dem Kohlenstoffatom in Position 3, 4, 5 oder 6 (wie oben beschrieben) verknüpft ist, ein (zumindest teilweise) anderes sensorisches Profil entsteht oder einzelne Sensorikdeskriptoren verstärkt, modifiziert oder schwächer ausgeprägt sein können. Die Komplexizität der sensorischen Profile der erfindungsgemäßen bzw. erfindungsgemäß zu verwendenden Verbindungen der Formel (A) ermöglicht vorteilhafterweise, dass durch die Wahl einer für eine gewünschte Anwendung besonders geeigneten Verbindungen der Formel (A) oder einer Mischung aus zwei oder mehr Verbindungen der Formel (A) (wie jeweils oben beschrieben) eine bestimmte Sensorik vorteilhaft hervorgehoben werden kann.

Eine erfindungsgemäß zu verwendende Verbindung der Formel (I) (wie oben beschrieben) weist neben typischen Grapefruitnoten (ähnlich denen des p-Menthenthiol-1,8) vorteilhafterweise auch schwefelig schalige Nuancen und Bucco- sowie schwarze Johannisbeernoten, aber auch Noten tropischer Früchte, auf.

Auch die erfindungsgemäß zu verwendende Verbindung der Formel (II) (wie oben beschrieben) weist durch seinen citrischen Grapefruit- und Limettencharakter, kombiniert mit lauchig zwiebeligen Schwefelnoten, vorteilhafterweise besonders interessante Aspekte auf.

Eine erfindungsgemäße bzw. eine erfindungsgemäß zu verwendende Verbindung der Formel (III) zeigt eine ähnliche sensorische Charakteristik wie eine Verbindung der Formel (I), ist aber hinsichtlich der Citrus-, tropische Früchte- und Bucco-Aspekte deutlich dominanter. Ebenso können durch eine Verbindung der Formel (III) vorteilhafterweise schwefelig staubige Noten von frischen Grapefruits bzw. Grapefruitschalen besonders lang anhaltend hervorgehoben werden.

Insbesondere eine erfindungsgemäße bzw. eine erfindungsgemäß zu verwendende Verbindung der Formel (IV) vermittelt vorteilhafterweise einen ausgesprochen intensiven Eindruck von schwarzer Johannisbeere.

Die erfindungsgemäßen bzw. erfindungsgemäß zu verwendenden Verbindungen der Formeln (III) und (IV) sind gegenüber dem Stand der Technik neue, erfindungsgemäße Verbindungen (siehe hierzu weiter unten).

Erfindungsgemäß bevorzugt ist eine Verwendung (wie oben beschrieben), wobei die Verbindung der Formel (A) bzw. eine, mehrere oder sämtliche Verbindungen der Formel (A) der Mischung ausgewählt ist bzw. jeweils unabhängig voneinander ausgewählt sind aus der Gruppe bestehend aus Verbindungen der Formeln (I) und (III)

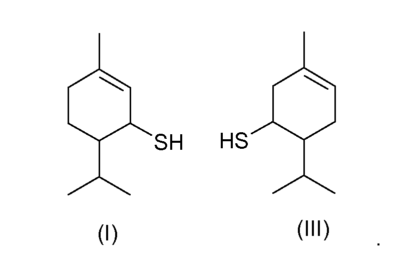

Besonders bevorzugt ist eine erfindungsgemäße Verwendung (wie oben beschrieben), wobei die Verbindung der Formel (A) bzw. eine, mehrere oder sämtliche Verbindungen der Formel (A) der Mischung ausgewählt ist bzw. jeweils unabhängig voneinander ausgewählt sind aus der Gruppe bestehend aus den Verbindungen der Formeln (la), (Ib), (Ic), (Id), (IIIa), (IIIb), (IIIc) und (IIId)

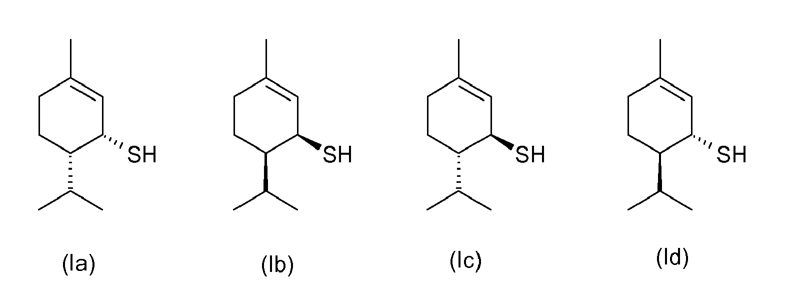

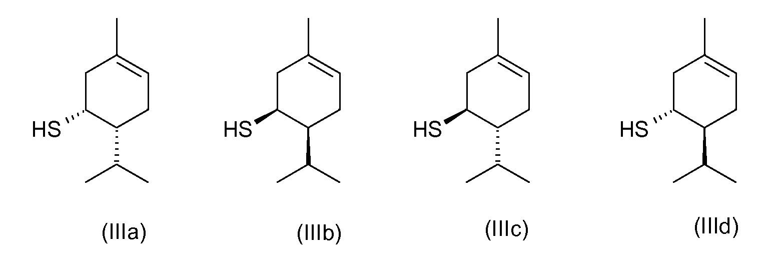

Erfindungsgemäß besonders bevorzugt zu verwendende Verbindungen der Formel (A) sind demnach Verbindungen der Formeln (I) und (III), insbesondere deren jeweilige Stereoisomere (Ia) bis (Id) und (IIIa) bis (IIId).

Besonders bevorzugt ist auch eine erfindungsgemäße Verwendung einer Mischung aus zwei oder mehr Verbindungen der Formel (A) (wie oben beschrieben), wobei die Mischung
eine oder mehrere Verbindungen der Formel (A), ausgewählt aus der Gruppe bestehend aus den Verbindungen der Formeln (Ia), (Ib), (Ic) und (Id), und/oder
eine oder mehrere Verbindungen der Formel (A), ausgewählt aus der Gruppe bestehend aus den Verbindungen der Formeln (IIIa), (IIIb), (IIIc) und (IIId),
vorzugsweise ausgewählt aus der Gruppe bestehend aus den Verbindungen der Formeln (Ib), (Id), (IIIb) und (IIId),
umfasst oder daraus besteht. Solche erfindungsgemäß bevorzugt zu verwendenden Mischungen weisen ein besonders vorteilhaftes und gesuchtes sensorisches Profil auf.

Die genannten Vorteile gelten insbesondere für eine weitere erfindungsgemäß bevorzugte Verwendung (wie oben beschrieben), wobei die Mischung eine oder mehrere Verbindungen der Formel (A), ausgewählt aus der Gruppe bestehend aus den Verbindungen der Formeln (la), (Ib), (Ic) und (Id), und eine oder mehrere Verbindungen der Formel (A), ausgewählt aus der Gruppe bestehend aus den Verbindungen der Formeln (IIIa), (IIIb), (IIIc) und (IIId) umfasst oder daraus besteht und
wobei das Gewichtsverhältnis der Gesamtmenge an Verbindungen der Formel (III) zur Gesamtmenge an Verbindungen der Formel (I) in der Mischung vorzugsweise im Bereich von 1 : 99 bis 20 : 80 liegt, vorzugsweise im Bereich von 2 : 98 bis 10 : 90, besonders bevorzugt im Bereich von 3 : 97 bis 8 : 92.

Ein weiterer Aspekt der vorliegenden Erfindung betrifft ein Verfahren zur Herstellung erfindungsgemäßer bzw. erfindungsgemäß zu verwendender Verbindungen der Formel
(A), umfassend folgende Schritte:
   (1) Umsetzen einer Verbindung der Formel (B) oder (C) 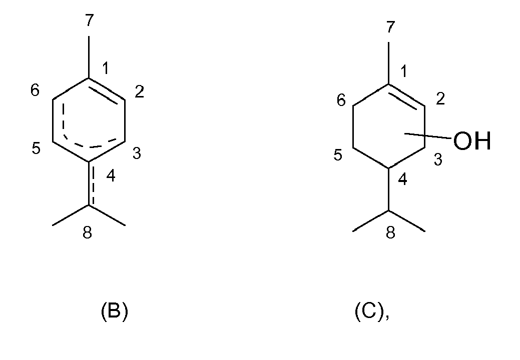 wobei in Formel (B) die gestrichelten Linien eine (zweite) Doppelbindung zwischen zwei benachbarten Kohlenstoffatomen innerhalb des Cyclohexanringes, das heißt zwischen den Kohlenstoffatomen der Position 6 und 5, 5 und 4 oder 4 und 3, oder außerhalb des Cyclohexanringes, das heißt zwischen den Kohlenstoffatomen in Position 4 und 8, bedeutet,
      wobei in Formel (C) (Piperitol und Hydroxy-Stellungsisomere) die OH-Gruppe an ein Kohlenstoffatom in einer der Positionen 3, 4, 5 oder 6 gebunden ist,
      mit Thioessigsäure (AcSH), gegebenenfalls in Gegenwart einer Zinkverbindung,
      und
   (2) alkalische Hydrolyse der in Schritt (1) erhaltenen Thioacetate mit einer Base, vorzugsweise ausgewählt aus der Gruppe bestehend aus LiOH, NaOH und Ca(OH)₂, und einem Lösungsmittel, vorzugsweise umfassend oder bestehend aus Wasser und einem mit Wasser (zumindest teilweise) mischbaren organischen Lösungsmittel, vorzugsweise ausgewählt aus der Gruppe bestehend aus THF, Dioxan, Aceton und Methanol.

Sofern Schritt (1) in Gegenwart einer (wasserfreien) Zinkverbindung durchgeführt wird, handelt es sich dabei vorzugsweise um ein Zinkhalogenid, weiter bevorzugt um Zinkiodid (Znl₂), Zinkbromid (ZnBr₂) oder Zinkchlorid (ZnCl₂).

Wie aus dem Stand der Technik bekannt (siehe zum Beispiel Tetrahedron: Asymmetry 2001, 12, 677-683 und Tetrahedron: Asymmetry 2000, 11, 3591-3607) sind Verbindungen der Formel (C) auch enantionmerenrein herstellbar. Bei Verwendung einer enantiomerenreinen Verbindung der Formel (C) in Schritt (1) können die erfindungsgemäß zu verwendenden Verbindungen der Formel (A), vorzugsweise die Verbindungen der Formeln (I) und (III), enantionmerenrein bzw. zumindest enantionmerenangereichert hergestellt werden.

So kann eine erfindungsgemäß bevorzugt zu verwendende Verbindung der Formel (I) (wie oben beschrieben) beispielsweise ausgehend von Piperiton oder Piperitol enantiomeren- bzw. stereoisomerenrein oder -angereichert hergestellt werden (vgl. Tetrahedron: Asymmetry 2001, 12, 677-683).

Eine erfindungsgemäß bevorzugt zu verwendende Mischung umfassend eine oder mehrere Verbindungen der Formel (I) und eine oder mehrere Verbindungen der Formel (III) (wie oben beschrieben) kann ausgehend von Piperitol, welches beispielsweise durch Reduktion von Piperiton erhältlich ist (Tetrahedron: Asymmetry 2001, 12, 677-683), in enantiomeren- bzw. stereoisomerenreiner oder -angereicherter Form hergestellt werden. Im Rahmen der vorliegenden Erfindung wurde in eigenen Versuchen beispielsweise ausgehend von enantiomerenreinem Piperitol durch Umsetzung mit Thioessigsäure in Gegenwart von wasserfreiem Zinkiodid und anschließender Verseifung des bei der Umsetzung gebildeten Thioacetats (nach chromatographischer Reinigung) eine Mischung hergestellt, die 57,5 Gew.-% an Verbindung der Formel (Id), 33,9 Gew.-% an Verbindung der Formel (Ib), 3,2 Gew.-% an Verbindung der Formel (IIId) und 2,3 Gew.-% an Verbindung der Formel (IIIb) umfasst.

Entsprechend wurde ausgehend von racemischem Piperiton über Piperitol nach chromatographischer Reinigung eine Mischung umfassend 28,9 Gew.-% an Verbindung der Formel (Id), 28,9 Gew.-% an Verbindung der Formel (Ic), 17,1 Gew.-% an Verbindung der Formel (Ib), 17,1 Gew.-% an Verbindung der Formel (Ia), 1,6 Gew.-% an Verbindung der Formel (IIId), 1,6 Gew.-% an Verbindung der Formel (IIIc), 1,2 Gew.-% an Verbindung der Formel (IIIb) und 1,2 Gew.-% an Verbindung (der Formel (IIIa) hergestellt.

Die erfindungsgemäß zu verwendenden Verbindungen der Formel (A) eignen sich vorteilhafterweise besonders gut für den Einsatz in der Ernährung oder dem Genuss dienenden Zubereitungen sowie als Bestandteil einer Riech- oder Aromastoffkomposition.

Dementsprechend betrifft die vorliegende Erfindung insbesondere eine erfindungsgemäße Verwendung (wie oben beschrieben) in einer Riech- oder Aromastoffkomposition oder einer der Ernährung oder dem Genuss dienenden Zubereitung.

Wie oben erwähnt sind die erfindungsgemäß zu verwendenden Verbindungen der Formeln (III) und (IV) im Stand der Technik noch nicht beschrieben. Ein weiterer Aspekt der vorliegenden Erfindung betrifft demnach eine Verbindung der Formel (III) oder (IV) 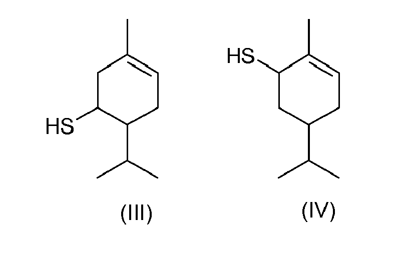 oder eine Mischung umfassend oder bestehend aus einer oder mehreren Verbindungen ausgewählt aus der Gruppe bestehend aus Verbindungen der Formeln (III) und (IV).

Besonders bevorzugt ist eine erfindungsgemäße Mischung (wie oben beschrieben), ferner umfassend eine oder mehrere Verbindungen ausgewählt aus der Gruppe bestehend aus Verbindungen der Formeln (I) und (II)

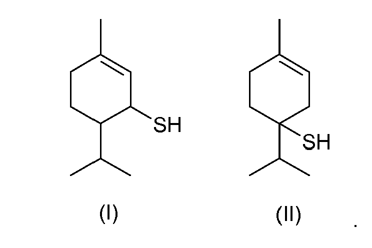

Für die besonders bevorzugten Verbindungen der Formeln (III) und (IV) bzw. besonders bevorzugten Mischungen sowie die darin enthaltenen Verbindungen der Formeln (I), (II), (III) und (IV) gilt das weiter oben Gesagte entsprechend.

Die erfindungsgemäß zu verwendenden bzw. erfindungsgemäßen Verbindungen der Formel (A) können vorteilhaft in Kombination mit weiteren Aroma- und/oder Riechstoffen zur Herstellung von Aroma- bzw. Riechstoffkompositionen eingesetzt werden. Hierfür können nicht nur einzelne Verbindungen der Formel (A), sondern auch eine Mischung mehrerer Verbindungen der Formel (A), insbesondere eine Mischung wie oben beschrieben, in Kombination mit (weiteren) Aromastoffen (z.B. mit Menthenthiolen), die keine Verbindungen der Formel (A) sind, eingesetzt werden. Zudem können die erfindungsgemäßen bzw. erfindungsgemäß zu verwendenden Verbindungen der Formel (A) oder erfindungsgemäße Riech- oder Aromastoffkompositionen (wie unten beschrieben) in aromatisierte oder zu aromatisierende Artikel, insbesondere in der Ernährung, der Mundpflege oder dem Genuss dienende Zubereitungen, eingearbeitet werden oder zur Parfümierung bestimmter Artikel verwendet werden.

Im Folgenden werden erfindungsgemäße Riech- oder Aromastoffkompositionen, erfindungsgemäße der Ernährung, dem Genuss oder der Mundpflege dienende Zubereitungen sowie erfindungsgemäße parfümierte Artikel beschrieben.

Ein weiterer Aspekt der vorliegenden Erfindung betrifft demnach eine Riech- oder Aromastoffkomposition, bestehend aus oder umfassend
(i) eine Verbindung der Formel (A) oder eine Mischung aus zwei oder mehr Verbindungen der Formel (A), wie weiter oben definiert, sowie
(ii) einen, zwei, drei oder mehr weitere Riech- und/oder Aromastoffe (vorzugsweise in sensorisch wirksamer Menge), wobei der weitere bzw. die weiteren Riech- und/oder Aromastoff(e) keine Verbindung(en) der Formel (A) ist bzw. sind,
mit der Maßgabe, dass die Riech- bzw. Aromastoffkomposition weder p-Menthenthiol-1,8 noch 1,8-*epi*-thio-*p*-Methan umfasst, und mit der weiteren Maßgabe, dass die Riech- oder Aromastoffkomposition bei Anwesenheit von Hexan und/oder Ethylacetat einen, zwei, drei oder mehr weitere Riech- und/oder Aromastoffe umfasst, wobei der weitere bzw. die weiteren Riech- und/oder Aromastoff(e) keine Verbindung(en) der Formel (A) ist bzw. sind. Wie weiter oben beschrieben, betrifft die vorliegende Erfindung keine Riech- oder Aromastoffmischung, die einer Mischung entspricht, wie sie in Flavour and Fragrance Journal 1993, 8, 289-294 beschrieben sind.

Erfindungsgemäße Riech- oder Aromastoffkompositionen (wie oben beschrieben) umfassen vorzugsweise insbesondere einen oder mehrere (weitere) sensorisch wirksame Stoffe wie zum Beispiel synthetische, natürliche oder naturidentische Aromastoffe oder Pflanzenextrakte. Das Mengenverhältnis von den erfindungsgemäßen bzw. erfindungsgemäß zu verwendenden Verbindungen der Formel (A) zu den weiteren Bestandteilen einer erfindungsgemäßen Riech- oder Aromastoffkomposition hängt naturgemäß stark von dem gewünschten sensorischen Gesamteindruck der Aromastoffkomposition ab und kann je nach gewünschtem Gesamteindruck vom Fachmann entsprechend eingestellt werden.

Erfindungsgemäß besonders bevorzugt ist eine Riech- oder Aromastoffkomposition (wie oben beschrieben), wobei die Gesamtmenge an Verbindungen der Formel (A) ausreicht, um in der Riech- bzw. Aromastoffkomposition den sensorischen Eindruck des weiteren bzw. eines, mehrerer oder sämtlicher weiteren Riech- und/oder Aromastoffe zu modifizieren und/oder zu verstärken.

Besonders bevorzugt ist zudem eine erfindungsgemäße Riech- oder Aromastoffkomposition (wie oben beschrieben), wobei
- der weitere bzw. einer, mehrere oder sämtliche weiteren Riechstoff(e) ausgewählt ist bzw. sind aus der Gruppe bestehend aus blumigen und fruchtigen Riechstoffen und/oder
- der weitere bzw. einer, mehrere oder sämtliche weiteren Aromastoff(e) ausgewählt ist bzw. sind aus der Gruppe bestehend aus blumigen und fruchtigen Aromastoffen.

Ein weiterer Aspekt der vorliegenden Erfindung betrifft die Verwendung einer erfindungsgemäßen Riech- oder Aromastoffkomposition (wie oben beschrieben) zum Vermitteln, Modifizieren und/oder Verstärken einer blumigen und/oder fruchtigen Geruchs- oder Geschmacksnote, insbesondere zum Vermitteln, Modifizieren und/oder Verstärken einer, zwei oder sämtlicher der Geruchs- oder Geschmacksnoten Grapefruit, tropische Früchte und schwarze Johannisbeere. Besonders bevorzugt sind demnach erfindungsgemäße Riech- oder Aromastoffkompositionen (wie oben beschrieben), die eine Menge an erfindungsgemäß zu verwendenden bzw. erfindungsgemäßen Verbindungen der Formel (A) enthalten, die ausreicht, um eine oder mehrere der Geruchs- und/oder Geschmacksnoten Grapefruit, tropische Früchte und schwarze Johannisbeere, vorzugsweise sämtliche der genannten Geruchs- und/oder Geschmacksnoten, zu vermitteln, zu modifizieren und/oder zu verstärken.

Erfindungsgemäße Verfahren zum Vermitteln, Modifizieren und/oder Verstärken eines Geruchs- und/oder Geschmacks werden weiter unten beschrieben.

Für die bevorzugt in einer erfindungsgemäßen Riech- oder Aromastoffkomposition enthaltenen Verbindungen der Formel (A) bzw. Mischungen gilt das weiter oben Gesagte entsprechend.

Die vorliegende Erfindung betrifft auch eine der Ernährung, dem Genuss oder der Mundpflege dienende Zubereitung, umfassend eine sensorisch wirksame Menge an
- einer Verbindung der Formel (A)
   oder
- einer Mischung aus zwei oder mehr Verbindungen der Formel (A),
   wie oben definiert,
   oder
- einer erfindungsgemäßen Verbindung der Formel (III) oder (IV) oder einer erfindungsgemäßen Mischung wie oben beschrieben,
   oder
- einer erfindungsgemäßen Riech- oder Aromastoffkomposition wie oben beschrieben
   sowie
- einen oder mehrere weitere zum Verzehr geeignete Bestandteile.

Für die bevorzugt enthaltenen Verbindungen der Formel (A) bzw. die bevorzugt enthaltenen erfindungsgemäßen Mischungen oder Riech- bzw. Aromastoffkompositionen gilt das weiter oben Gesagte entsprechend.

Die erfindungsgemäßen Zubereitungen weisen vorteilhafterweise besonders überraschende additive und ausgewogene Geschmackseindrücke sowie ein natürliches, komplexeres und authentischeres Geschmacksprofil auf.

Auch die erfindungsgemäßen Riech- und Aromastoffkompositionen (wie oben beschrieben) weisen überraschenderweise besonders vorteilhafte additive und ausgewogene Geschmackseindrücke auf, gewinnen mehr Körper und bekommen ein natürlicheres, komplexeres und authentischeres Geschmacksprofil.

Es ist zudem besonders vorteilhaft, dass die erfindungsgemäßen bzw. erfindungsgemäß zu verwendenden Verbindungen der Formel (A) außerordentlich intensiv, haftfest und komplex sind. Daher sind sie sehr ergiebig, können in sehr niedrigen Dosierungen (zum Teil im ppb-Bereich und darunter) in den Zubereitungen eingesetzt werden und zeigen eine lang anhaltende Wirkung.

Daneben wurde gefunden, dass die erfindungsgemäßen bzw. erfindungsgemäß zu verwendenden Verbindungen der Formel (A) nicht nur in der Lage sind, einer Riech- oder Aromastoffkomposition (insbesondere einer solchen wie oben beschrieben) wertvolle und spezielle Geschmacks- bzw. Geruchsnoten hinzuzufügen.

Insbesondere bei erfindungsgemäßen Aromastoffkompositionen wurde im Rahmen eigener Untersuchungen ein geschmacksverstärkender Effekt (Booster bzw. Enhancer) durch die enthaltene(n) erfindungsgemäß zu verwendende(n) Verbindung(en) der Formel (A) beobachtet, der sich insbesondere durch einen insgesamt stärkeren und vorzugsweise abgerundeteren Geschmackseindruck auszeichnet.

Die erfindungsgemäßen bzw. erfindungsgemäß zu verwendenden Verbindungen der Formel (A) sind vorteilhafterweise besonders gut zur Einarbeitung in Aroma- bzw. Riechstoffkompositionen und der Ernährung, dem Genuss oder der Mundpflege dienenden Zubereitungen geeignet, in denen fruchtige und/oder exotisch fruchtige Noten erwünscht sind oder sogar dominieren (sollen). Insbesondere zählen hierzu die Geschmacksrichtungen Erdbeere, Himbeere, Brombeere, Holunderbeere, Stachelbeere, rote und schwarze Johannisbeere, Heidelbeere, Blaubeere, Preiselbeere, Kirsche, Birne, Apfel, Rhabarber, Traube, Pflaume, Mirabelle, Pfirsich, Aprikose, Nektarine, Quitte, Kiwi, Banane, Zitrone, Orange, Apfelsine, Mandarine, Clementine, Grapefruit, Pampelmuse, Limone, Limette, Kumquat, Tangor, Tangelo, Sternfrucht, Litschie, Ananas, Guave, Papaya, Maracuja und Mango.

In einer erfindungsgemäßen Aromastoffkomposition (wie oben beschrieben) ist der weitere bzw. sind einer, mehrere oder (vorzugsweise) sämtliche der weiteren Aromastoffe, besonders bevorzugt zwei, drei, vier, fünf, sechs, sieben, acht, neun oder zehn weiteren Aromastoffe, ausgewählt aus der Gruppe bestehend aus:
Acetophenon, Allylcapronat, alpha-lonon, beta-lonon, Anisaldehyd, Anisylacetat, Anisylformiat, Benzaldehyd, Benzothiazol, Benzylacetat, Benzylalkohol, Benzylbenzoat, beta-lonon, Buttersäure, Butylbutyrat, Butylcapronat, Butylidenphthalid, Carvon, Camphen, Caryophyllen, Cineol, Cinnamylacetat, Citral, Citronellol, Citronellal, Citronellylacetat, Cyclohexylacetat, Cymol, Damascon, Decalacton, Essigsäure, Dihydrocumarin, Dimethylanthranilat, Dimethylanthranilat, Dodecalacton, Ethoxyethylacetat, Ethylbuttersäure, Ethylbutyrat, Ethylcaprinat, Ethylcapronat, Ethylcrotonat, Ethylfuraneol, Ethylguajakol, Ethylisobutyrat, Ethylisovalerianat, Ethyllactat, Ethylmethylbutyrat, Ethylpropionat, Eucalyptol, Eugenol, Ethylheptylat, 4-(p-Hydroxyphenyl)-2-butanon, gamma-Decalacton, Geraniol, Geranylacetat, Geranylacetat, Grapefruitaldehyd, Methyldihydrojasmonat (z.B. Hedion^{®}), Heliotropin, 2-Heptanon, 3-Heptanon, 4-Heptanon, trans-2-Heptenal, cis-4-Heptenal, trans-2-Hexenal, cis-3-Hexenol, trans-2-Hexensäure, trans-3-Hexensäure, cis-2-Hexenylacetat, cis-3-Hexenylacetat, cis-3-Hexenylcapronat, trans-2-Hexenylcapronat, cis-3-Hexenylformiat, cis-2-Hexylacetat, cis-3-Hexylacetat, trans-2-Hexylacetat, cis-3-Hexylformiat, para-Hydroxybenzylaceton, Isoamylalkohol, Isoamylisovalerianat, Isobutylbutyrat, Isobutyraldehyd, Isoeugenolmethylether, Isopropylmethylthiazol, Laurinsäure, Leavulinsäure, Linalool, Linalooloxid, Linalylacetat, Menthol, Menthofuran, Methylanthranilat, Methylbutanol, Methylbuttersäure, 2-Methylbutylacetat, Methylcapronat, Methylcinnamat, 5-Methylfurfural, 3,2,2-Methylcyclopentenolon, 6,5,2-Methylheptenon, Methyldihydrojasmonat, Methyljasmonat, 2-Methylmethylbutyrat, 2-Methyl-2-Pentenolsäure, Methylthiobutyrat, 3,1-Methylthiohexanol, 3-Methylthiohexylacetat, Nerol, Nerylacetat, trans,trans-2,4-Nonadienal, 2,4-Nonadienol, 2,6-Nonadienol, 2,4-Nonadienol, Nootkaton, delta Octalacton, gamma Octalacton, 2-Octanol, 3-Octanol, 1,3-Octenol, 1-Octylacetat, 3-Octylacetat, Palmitinsäure, Paraldehyd, Phellandren, Pentandion, Phenylethylacetat, Phenylethylalkohol, Phenylethylalkohol, Phenylethylisovalerianat, Piperonal, Propionaldehyd, Propylbutyrat, Pulegon, Pulegol, Sinensal, Sulfurol, Terpinen, Terpineol, Terpinolen, 8,3-Thiomenthanon, 4,4,2-Thiomethylpentanon, Thymol, delta-Undecalacton, gamma-Undecalacton, Valencen, Valeriansäure, Vanillin, Acetoin, Ethylvanillin, Ethylvanillinisobutyrat (= 3-Ethoxy-4-isobutyryloxybenzaldehyd), 2,5-Dimethyl-4-hydroxy-3(2H)-furanon und dessen Abkömmlinge (dabei vorzugsweise Homofuraneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), Cumarin und Cumarin-Abkömmlinge, gamma-Lactone (dabei vorzugsweise gamma-Undecalacton, gamma-Nonalacton, gamma-Decalacton), delta-Lactone (dabei vorzugsweise 4-Methyldeltadecalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat, 4-(p-Hydroxyphenyl)-2-butanon, 1,1-Dimethoxy-2,2,5-trimethyl-4-hexan, 2,6-Dimethyl-5-hepten-1-al und Phenylacetaldeh y d , 2-Methyl-3-(methylthio)furan, 2-Methyl-3-furanthiol, bis(2-Methyl-3-furyl)disulfid, Furfurylmercaptan, Methional, 2-Acetyl-2-thiazolin, 3-Mercapto-2-pentanon, 2,5-Dimethyl-3-furanthiol, 2,4,5-Trimethylthiazol, 2-Acetylthiazol, 2,4-Dimethyl-5-ethylthiazol, 2-Acetyl-1-pyrrolin, 2-Methyl-3-ethylpyrazin, 2-Ethyl-3,5-dimethylpyrazin, 2-Ethyl-3,6-dimethylpyrazin, 2,3-Diethyl-5-methylpyrazin, 3-Isopropyl-2-methoxypyrazin, 3-Isobutyl-2-methoxypyrazin, 2-Acetylpyrazin, 2-Pentylpyridin, (E,E)-2,4-Decadienal, (E,E)-2,4-Nonadienal, (E)-2-Octenal, (E)-2-Nonenal, 2-Undecenal, 12-Methyltridecanal, 1-Penten-3-on, 4-Hydroxy-2,5-dimethyl-3(2H)-furanon, Guajakol, 3-Hydroxy-4,5-dimethyl-2(5H)-furanon, 3-Hydroxy-4-methyl-5-ethyl-2(5H)-furanon, Zimtaldehyd, Zimtalkohol, Methylsalicylat, Isopulegol sowie deren Stereoisomere, Enantiomere, Diastereomere sowie gegebenenfalls cis/trans-Isomere.

Reaktionsmischungen, wie sie gemäß Fragrance Journal 1993, 8, 289-294 durch Umsetzung ausgehend jeweils von Limonen, alpha-Pinen, alpha-Terpinen, gamma-Terpinen bzw. 3-Caren mit Schwefelwasserstoff in Gegenwart von Aluminiumtrichlorid erhältlich sind, und sonstige dort offenbarte Mischungen sind keine Aromastoffkompositionen im Sinne der vorliegenden Erfindung und insoweit vom Schutzumfang ausgenommen (vgl. oben).

Aufgrund ihrer typischen Citrus- und Grapefruitnoten eignen sich die erfindungsgemäßen bzw. die erfindungsgemäß zu verwendenden, insbesondere die vorangehend als bevorzugt beschriebenen, Verbindungen der Formel auch besonders gut für den Einsatz in (erfindungsgemäßen) Riechstoffkompositionen (wie oben beschrieben). Solche Riechstoffkompositionen sind vorzugsweise Parfümkompositionen (Parfümöle). Dabei lassen sich bereits in sehr geringen Dosierungen der erfindungsgemäßen bzw. erfindungsgemäß zu verwendenden Verbindungen der Formel (A) feine, schalige Grapefruitnoten erzielen, wobei der geruchliche Gesamteindruck auffallend harmonisiert, die Ausstrahlung wahrnehmbar erhöht und die Haftfestigkeit der Parfümkompositionen deutlich verstärkt wird.

Die erfindungsgemäßen bzw. die erfindungsgemäß zu verwendenden Verbindungen der Formel (A) können entweder einzeln oder in einer beliebigen Mischung (wie jeweils oben beschrieben) in einer Vielzahl von Produkten verwendet werden. Besonders vorteilhaft lassen sie sich mit anderen Riechstoffen in unterschiedlichen Mengenverhältnissen zu neuartigen Parfümkompositionen kombinieren.

Beispiele für Riechstoffe, mit denen die erfindungsgemäß zu verwendenden Verbindungen der Formel (A) vorteilhaft kombiniert werden können und die demnach bevorzugt in einer einfindungsgemäßen Riechstoffkomposition (wie oben beschriebene) enthalten sind, finden sich z.B. in S. Arctander, Perfume and Flavor Materials, Vol. I und II, Montclair, N. J., 1969, Selbstverlag oder H. Surburg, J. Panten, Common Fragrance and Flavor Materials, 5th. Ed., Wiley-VCH, Weinheim 2006.

Im Einzelnen seien genannt:
Extrakte aus natürlichen Rohstoffen wie Etherische Öle, Concretes, Absolues, Resine, Resinoide, Balsame, Tinkturen wie z. B.

Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl, sowie Fraktionen davon, bzw. daraus isolierten Inhaltsstoffen;
Einzel-Riechstoffe aus der Gruppe
der Kohlenwasserstoffe, wie z. B. 3-Caren; α- Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole wie z. B. Hexanol; Octanol; 3-Octanol; 2,6-Dimethylheptanol; 2-Methyl-2-heptanol; 2-Methyl-2-octanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z. B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal; 2,6,10-Trimethyl-5,9-undecadienal; Heptanaldiethylacetal; 1,1-Dimethoxy-2,2,5-trimethyl-4-hexen; Citronellyloxyacetaldehyd;
der aliphatischen Ketone und deren Oxime wie z. B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on;
der aliphatischen schwefelhaltigen Verbindungen wie z. B. 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Tridecensäurenitril; 2,12-Tridecadiensäurenitril; 3,7-Dimethyl-2,6-octadiensäurenitril; 3,7-Dimethyl-6-octensäurenitril;
der aliphatischen Carbonsäuren und deren Ester wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenylisobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;
der acyclischen Terpenalkohole wie z. B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate, 3-Methyl-2-butenoate;
der acyclischen Terpenaldehyde und -ketone wie z. B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole wie z. B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate, 3-Methyl-2-butenoate;
der cyclischen Terpenaldehyde und -ketone wie z. B. Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on; Nootkaton; Dihydronootkaton; alpha-Sinensal; beta-Sinensal; Acetyliertes Cedernholzöl (Methylcedrylketon);
der cyclischen Alkohole wie z.B. 4-tert.-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha,3,3-Trimethylcyclohexylmethanol; 2-Methyl-4-(2,2,3-trimethyl-3-cyclopent-1-yl)butanol; 2-Methyl-4-(2,2,3-trimethyl-3-cyclopent-1-yl)-2-buten-l-ol; 2-Ethyl-4-(2,2,3-trimethyl-3-cyclopent-1-yl)-2-buten-l-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone wie z.B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 7-Cyclohexadecen-1-on; 8-Cyclohexadecen-1-on; 9-Cycloheptadecen-1-on; Cyclopentadecanon; Cyclohexadecanon;
der cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z. B. 1-(3,3-Dimethylcyclohexyl)-4-penten-1-on; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B. 2-tert-Butylcyclohexylacetat; 4-tert-Butylcyclohexylacetat; 2-tert-Pentylcyclohexylacetat; 4-tert-Pentylcyclohexylacetat; Decahydro-2-naphthylacetat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Carbonsäuren wie z. B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der araliphatischen Alkohole wie z.B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-l-ol; 4-Methoxybenzylalkohol; 1-(4-Isopropylphenyl)ethanol;
der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxane; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon; Benzophenon; 1,1,2,3,3,6-Hexamethyl-5-indanylmethylketon; 6-tert.-Butyl-1, 1 -dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 5-Phenyl-3-methyl-2-pentensäurenitril; 5-Phenyl-3-methylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

Reaktionsmischungen, wie sie gemäß Fragrance Journal 1993, 8, 289-294 durch Umsetzung ausgehend jeweils von Limonen, alpha-Pinen, alpha-Terpinen, gamma-Terpinen bzw. 3-Caren mit Schwefelwasserstoff in Gegenwart von Aluminiumtrichlorid erhältlich sind, und sonstige dort offenbarte Mischungen sind auch keine (erfindungsgemäßen) Parfümkompositionen im Sinne der vorliegenden Erfindung und insoweit vom Schutzumfang ausgenommen (vgl. oben).

In erfindungsgemäßen Parfümkompositionen (Parfümölen) beträgt die eingesetzte Gesamtmenge der Verbindungen der Formel (A) vorzugsweise 0,00001 Gew.-% (entsprechend 0,1 ppm) bis 5 Gew.-%, bevorzugt 0,00005 Gew.-% (entsprechend 0,5 ppm) bis 1 Gew.-%, weiter bevorzugt 0,0001 Gew.-% (entsprechend 1 ppm) bis 0,2 Gew.-% (entsprechend 2000 ppm), am meisten bevorzugt 0,0001 Gew.-% (entsprechend 1 ppm) bis 0,02 Gew.-% (entsprechend 200 ppm), jeweils bezogen auf das Gesamtgewicht der Parfümkomposition.

In einer bevorzugten Ausgestaltung einer erfindungsgemäßen Riech- oder Aromastoffkomposition sind die erfindungsgemäß zu verwendenden Verbindungen der Formel (A) vorzugsweise mit einem oder mehreren, besonders bevorzugt mit zwei, drei, vier, fünf, sechs oder mehr, bevorzugt blumigen und/oder fruchtigen (weiteren) Riechstoffen kombiniert.

Dabei wird durch die erfindungsgemäß enthaltenen Verbindungen der Formel (A) vorteilhafterweise (zumindest teilweise) eine geruchliche Verstärkung der blumigen Riech- bzw. Aromastoffe erreicht.

Blumige Riech- oder Aromastoffe, mit denen die erfindungsgemäß zu verwendenden Verbindungen der Formel (A) (insbesondere in erfindungsgemäßen Riech- oder Aromastoffkompositionen) vorteilhaft kombiniert werden können, werden vorzugsweise ausgewählt aus der Gruppe bestehend aus:
Hydroxycitronellal, Methoxycitronellal, Cyclamenaldehyd [2-Methyl-3-(4-isopropylphenyl)propanal], 1-(4-Isopropyl-cyclohexyl)ethanol (Mugetanol^{®}), 4-tert.-Butyl-a-methyldihydrozimtaldehyd (Lilial^{®}), cis-Hexahydrocuminylalkohol (Mayol^{®}), 3-[4-(1,1-Dimethylethyl)phenyl]propanal (Bourgeonal^{®}), 2,2-Dimethyl-3-(3-methylphenyl)propanol (Majantol^{®}), 3-Methyl-3-(3-methylbenzyl)-butan-2-ol, 2-Isobutyl-4-methyltetrahydro-2H-pyran-4-ol (Florosa^{®}), 2-Methyl-3-(3,4-methylendioxyphenyl)propanal (Heliofolal^{®}), 4-(4-Hydroxy-4-methylpentyl)-3-cyclohexencarbaldehyd (Lyral^{®}), 4-(Octahydro-4,7-methano-5H-inden-5-yliden-butanal (Dupical^{®}), Vernaldehyd, 4-(4-Methyl-3-penten-1-yl)-3-cyclohexencarbaldehyd (Vertomugal^{®}), Octahydro-5-(4-methoxybutyliden)-4,7-methano-1H-inden (Mugoflor^{®}), 2,6-Dimethyl-2-heptanol (Freesiol^{®}), 1-Ethyl-1-methyl-3-phenylpropanol (Phemec^{®}), 2,2-Dimethyl-3-phenyl-1-propanol (Muguet alcohol), Profarnesol, Dihydrofarnesol, Farnesol, Nerolidol, Hydroxycitronellaldimethylacetal, Hexylbenzoat, Geraniol, Nerol, Linalool, Tetrahydrogeraniol, Tetrahydrolinalool, Ethyllinalool, Geranyltiglinat, Phenethylalkohol (2-Phenylethylalkohol), Citronellol, Rosenoxid, 2-Methyl-5-phenylpentanol (Rosaphen), 3-Methyl-5-phenylpentanol (Phenoxanol), Methyldihydrojasmonat (Hedion^{®}, Hedione^{®} high cis), 2-Heptylcyclopentanon (Projasmon P), cis-Jasmon, Dihydrojasmon, Zimtalkohol (3-Phenyl-2-propen-l-ol), Dihydrozimtalkohol (3-Phenylpropanol), 2-Methyl-4-phenyl-1,3-dioxolan (Jacinthaflor^{®}) und Dihydromyrcenol (2,6-Dimethyl-7-octen-2-ol).

Zudem sind erfindungsgemäße bzw. erfindungsgemäß zu verwendende Verbindungen der Formel (A) vorteilhafterweise dazu geeignet, fruchtige Riech- oder Aromastoffe, insbesondere fruchtige Riechstoffe, hinsichtlich ihres Geruchs zu verstärken.

Fruchtige Riech- oder Aromastoffe, mit denen die erfindungsgemäß zu verwendenden Verbindungen der Formel (A) vorteilhaft kombiniert werden können, und die demnach besonders bevorzugte (weitere) Riech- oder Aromastoffe einer erfindungsgemäßen Riech- oder Aromastoffkompositionen sind, sind vorzugsweise ausgewählt der Gruppe bestehend aus:
2-Methyl-buttersäureethylester, 4-(p-Hydroxyphenyl)-2-butanon, Ethyl-3-methyl-3-phenylglycidat, Buttersäureisoamylester, Essigsäureisoamylester, Essigsäure-n-butylester, Buttersäureethylester, 3-Methyl-buttersäureethylester, n-Hexansäureethylester, n-Hexansäureallylester, Ethyl-2-trans-4-cis-decadienoat, 1,1-Dimethoxy-2,2,5-trimethyl-4-hexan, 2,6-Dimethyl-5-hepten-1-al, gamma-Undecalacton, gamma-Nonalacton, Hexanal, 3Z-Hexenal, n-Decanal, n-Dodecanal, Citral, Vanillin, Ethylvanillin, Maltol, Ethylmaltol und deren Mischungen.

Erfindungsgemäße Parfümöle (wie oben beschrieben) können in flüssiger Form, unverdünnt oder mit einem Lösungmittel verdünnt für Parfümierungen (von Artikeln) eingesetzt werden. Geeignete Lösungsmittel hierfür sind z.B. Ethanol, Isopropanol, Diethylenglycolmonoethylether, Glycerin, Propylenglycol, 1,2-Butylenglycol, Dipropylenglycol, Diethylphthalat, Triethylcitrat und Isopropylmyristat.

Des Weiteren können die erfindungsgemäßen Parfümöle (wie oben beschrieben) an einem Trägerstoff adsorbiert sein, der sowohl für eine feine Verteilung der Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate und Gasbeton oder organische Materialien wie Hölzer und Cellulose-basierende Stoffe sein.

Erfindungsgemäße Parfümöle (wie oben beschrieben) können auch mikroverkapselt, sprühgetrocknet, als Einschluss-Komplexe oder als Extrusions-Produkte vorliegen und in dieser Form vorzugsweise einem zu parfümierenden Produkt hinzugefügt werden.

Gegebenenfalls können die Eigenschaften der derart modifizierten Parfümöle durch sogenanntes "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden.

Eine Mikroverkapselung erfindungsgemäßer Parfümöle kann beispielsweise durch das sogenannte Koazervationsverfahren mit Hilfe von Kapselmaterialien z.B. aus polyurethan-artigen Stoffen oder Weichgelatine, erfolgen. Die sprühgetrockneten Parfümöle können beispielsweise durch Sprühtrocknung einer das Parfümöl enthaltenden Emulsion, bzw. Dispersion hergestellt werden, wobei als Trägerstoffe modifizierte Stärken, Proteine, Dextrin und pflanzliche Gummen verwendet werden können. Einschluss-Komplexe können z.B. durch Mischen von erfindungsgemäßen Parfümölen und Cyclodextrinen oder Harnstoffderivaten in einem geeigneten Lösungsmittel, z.B. Wasser, hergestellt werden. Extrusions-Produkte können durch Verschmelzen der (erfindungsgemäßen) Parfümöle mit einem geeigneten extrudierbaren Stoff und durch Extrusion mit nachfolgender Erstarrung, gegebenenfalls in einem geeigneten Lösungsmittel (z.B. Isopropanol) erfolgen.

Die erfindungsgemäßen Parfümöle (wie oben beschrieben) können vorteilhafterweise in konzentrierter Form, in Lösungen oder in wie oben beschriebener modifizierter Form für die Herstellung von parfümierten Artikeln verwendet werden.

Dementsprechend betrifft die vorliegende Erfindung auch einen parfümierten Artikel, umfassend eine erfindungsgemäße Riech- oder Aromastoffkomposition, vorzugsweise eine erfindungsgemäße Parfümkomposition (Parfümöl). Bevorzugte parfümierte bzw. zu parfümierende Artikel sind Parfüm-Extraits, Eau de Parfums, Eau de Toilettes, Rasierwässer, Eau de Colognes, Pre-shave-Produkte, Splash-Colognes und parfümierte Erfrischungstücher saure, alkalische und neutrale Reinigungsmittel, wie z.B. Fußbodenreiniger, Fensterglasreiniger, Geschirrspülmittel, Bad- und Sanitärreiniger, Scheuermilch, feste und flüssige WC-Reiniger, pulver- und schaumförmige Teppichreiniger, flüssige Waschmittel, pulverförmige Waschmittel, Wäschevorbehandlungsmittel wie Bleichmittel, Einweichmittel und Fleckenentferner, Wäscheweichspüler, Waschseifen, Waschtabletten, Desinfektionsmittel, Oberflächendesinfektionsmittel sowie Luftverbesserer in flüssiger, gelartiger oder auf einem festen Träger aufgebrachter Form, Aerosolspray, Wachse und Polituren wie Möbelpolituren, Fußbodenwachse, Schuhcremes, sowie Körperpflegemittel wie z.B. feste und flüssige Seifen, Duschgele, Shampoos, Rasierseifen, Rasierschäume, Badeöle, kosmetische Emulsionen vom Öl-in-Wasser-, Wasser-in-Öl- und Wasser-in-Öl-in-Wasser-Typ wie z.B. Hautcremes- und -lotionen, Gesichtscremes und -lotionen, Sonnenschutzcremes- und -lotionen, After-sun-cremes und -lotionen, Handcremes und - lotionen, Fußcremes und -lotionen, Enthaarungscremes und -lotionen, After-shave-Cremes und -lotionen, Bräunungscremes und -lotionen, Haarpflegeprodukte wie z.B. Haarsprays, Haargele, festigende Haarlotionen, Haarspülungen, permanente und semipermanente Haarfärbemittel, Haarverformungsmittel wie Kaltwellen- und Haarglättungsmitteln, Haarwässer, Haarcremes und -lotionen, Deodorantien und Antiperspirantien wie z.B. Achselsprays, Roll-ons, Deosticks, Deocremes, sowie Produkte der dekorativen Kosmetik wie z.B. Lidschatten, Nagellacke, Make-ups, Lippenstifte, Mascara sowie Kerzen, Lampenöle, Räucherstäbchen, Insektizide, Repellentien und Treibstoffe. Erfindungsgemäße der Ernährung, dem Genuss oder der Mundpflege dienende Zubereitungen (wie oben beschrieben) enthalten bevorzugt 0,000000001 Gew.-% (0,00001 ppm = 0,01 ppb) bis 0,05 Gew.-% (500 ppm), bevorzugt 0,0000001 Gew.-% (0,001 ppm = 1 ppb) bis 0,005 Gew.-% (50 ppm), besonders bevorzugt 0,0000002 Gew.-% (0,002 ppm = 2 ppb) bis 0,001 Gew.-% (10 ppm) an erfindungsgemäßen bzw. erfindungsgemäß zu verwendenden Verbindungen der Formel (A), bezogen auf das Gesamtgewicht der Zubereitung.

Weitere übliche Grund-, Hilfs- und Zusatzstoffe für Nahrungs- oder Genussmittel können in Mengen von bis zu 99,999999 Gew.-%, vorzugsweise 10 bis 95 Gew.-%, bezogen auf das Gesamtgewicht der Zubereitung, enthalten sein. Ferner können die (erfindungsgemäßen) Zubereitungen Wasser in einer Menge bis zu 99,999999 Gew.-%, vorzugsweise 5 bis 80 Gew.-%, bezogen auf das Gesamtgewicht der Zubereitung, umfassen.

Die der Ernährung oder dem Genuss dienenden erfindungsgemäßen Zubereitungen sind vorzugsweise beispielsweise Backwaren (z.B. Brot, Trockenkekse, Kuchen, sonstiges Gebäck), Süßwaren (z.B. Schokoladen, Fruchtgummi, Hart- und Weichkaramellen, Kaugummi, Lakritze), alkoholische oder nicht-alkoholische Getränke (z.B. Kaffee, Tee, Kakao, kakaohaltige Getränke, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke, Fleischprodukte (z.B. Schinken, Frischwurst- oder Rohwurstzubereitungen), Eier oder Eiprodukte (Trockenei, Eiweiß, Eigelb), Getreideprodukte (z.B. Frühstückscerealien, Müsliriegel), Milchprodukte (z.B. Milchgetränke, Milcheis, Joghurt, Kefir, Frischkäse, Weichkäse, Hartkäse, Trockenmilchpulver, Molke, Butter, Buttermilch), Fruchtzubereitungen (z.B. Konfitüren, Fruchteis, Fruchtsoßen), Gemüsezubereitungen (z.B. Ketchup, Soßen, Trockengemüse), Knabberartikel (z.B. gebackene oder frittierte Kartoffelchips oder Kartoffelteigprodukte, Extrudate auf Mais- oder Erdnussbasis), Produkte auf Fett- und Ölbasis oder Emulsionen derselben (z.B. Mayonnaise, Remoulade, Dressings), Fertiggerichte und Suppen, Gewürze, Würzmischungen sowie insbesondere Aufstreuwürzungen (Seasonings), die im Snackbereich Anwendung finden. Die (erfindungsgemäßen) Zubereitungen im Sinne der vorliegenden Erfindung können auch als Halbfertigware zur Herstellung weiterer der Ernährung oder dem Genuss dienender Zubereitungen dienen. Die (erfindungsgemäßen) Zubereitungen im Sinne der vorliegenden Erfindung können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen.

Erfindungsgemäße der Ernährung oder dem Genuss dienende Zubereitungen sind bevorzugt alkoholische oder nicht-alkoholische Getränke, die zudem vorzugsweise einen oder mehrere übliche Zusätze wie Fruchtbestandteile, im Lebensmittelbereich zugelassene anorganische oder organische Säuren, Antioxidantien, Trübungsmittel, Beschwerungsmittel, Süßungsmittel, Aromen, Farbstoffe, Verdickungsmittel, sogenannte "funktionelle" Zutaten und Konservierungsstoffe umfassen.

Als Fruchtbestandteile werden hier vor allem Fruchtaromen, Fruchtsäfte, Fruchtpürees und Fruchtsaftkonzentrate verstanden. Fruchtsäfte bzw. Fruchtsaftkonzentrate, die verwendet werden können, sind solche auf Basis von Zitrusfrüchten, beispielsweise Orange, Zitrone, Grapefruit und Mandarine, und anderen Früchten, beispielsweise Apfel, Birne, Traube, Aprikose und Ananas. Weiterhin können Fruchtsäfte und Fruchtsaftkonzentrate aus Beerenobst, wie Brombeere, Stachelbeere, Johannisbeere, Heidelbeere, Erdbeere und Himbeere verwendet werden. Weiterhin können Fruchtsäfte und Fruchtsaftkonzentrate aus exotischen Früchten, wie beispielsweise Guave, Papaya, Maracuja, Mango und Banane verwendet werden.

Als Süßungsmittel werden üblicherweise Zucker (insbesondere Saccharose), Süßstoffe, Zuckeraustauschstoffe und Süßungsmittelzusammensetzungen, sowie Mischungen davon eingesetzt.

Geeignete Süßstoffe sind insbesondere Acesulfam-K, Aspartam, Cyclamat (sowie dessen Na- und Ca-Salze), Neohesperidindihydrochalcon, Sucralose und Saccharin (sowie dessen Na-, K- und Ca-Salze). Pflanzliche Süßstoffe können ebenfalls verwendet werden, wie beispielsweise Glycyrrhicin und Thaumatin. Besonders bevorzugt sind Acesulfam-K, Aspartam, Cyclamat, Na- Cyclamat, Saccharin, Na-Saccharin und Sucralose.

Geeignete Zuckeraustauschstoffe sind Zuckeralkohole wie Isomatitol (E 953), Lactitol (E 966), Maltitol, Manitol (E 421), Sorbitol (E 420), Xylitol (E 967) sowie Gemische daraus. Weiterhin können als Süßungsmittel Mischungen aus synthetischem Süßstoff und entaromatisierten oder nicht entaromatisierten konzentrierten Fruchtzubereitungen verwendet werden.

Geeignete Emulgatoren in erfindungsgemäßen Getränken sind beispielsweise Gummi arabicum oder modifizierte Stärken, dabei bevorzugt Stärkenatriumoctenylsuccinat (E 1450).

Erfindungsgemäße alkoholische oder nicht-alkoholische Getränke können zudem Terpen-Öle enthalten. Bevorzugte Terpen-Öle im Rahmen der vorliegenden Erfindung umfassen oder bestehen aus Orangen-, Zitronen- und/oder Grapefruitöl oder daraus erhältlichen Fraktionen, bevorzugt Limonen (insbesondere d-Limonen) und/oder Orangenöl-Terpenen.

Bevorzugt einzusetzende Beschwerungsmittel sind SAIB (Saccharoseacetat-Isobutyrat, E 444), Estergum (E 445) sowie in eingeschränktem Maße Dammar Gum und BVO's (brominated vegetable oils). Diese ermöglichen stabile Getränketrübungen bei Emulsionsgetränken wie beispielsweise in Limonaden oder Fruchtsaftgetränken.

Ein erfindungsgemäßes Getränk enthält regelmäßig eine oder mehrere Genusssäuren. Bevorzugte Genusssäuren sind Citronensäure, Weinsäure, Milchsäure, Phosphorsäure und Äpfelsäure. Der pH-Wert erfindungsgemäßer Emulsionsgetränke liegt regelmäßig im Bereich von 3 bis 5.

Weiterhin können erfindungsgemäße alkoholische oder nicht-alkoholische Getränke Hydrokolloide als Verdickungsmittel enthalten. Geeignete Verdickungsmittel sind Carboxymethylcellulose, Xanthan, Johannisbrotkernmehl, Gellan, Guarkemmehl, Gummi arabicum, Carragen, Alginsäure, Alginate, Pektin sowie Mischungen daraus.

Erfindungsgemäße alkoholische oder nicht-alkoholische Getränke können weiterhin sogenannte "funktionelle" Zutaten enthalten, wie beispielsweise Vitamine (A, B, C, D, E, K), Mineralstoffe, Kräuterextrakte, Ballaststoffe, prebiotische Zutaten, Aminosäuren, Taurin und/oder Coffein.

Erfindungsgemäße alkoholische oder nicht-alkoholische Getränke können mit Lebensmittelfarbstoffen und/oder färbenden Lebensmittel eingefärbt werden (z.B. beta-Carotin).

Geeignete Konservierungsstoffe sind beispielsweise, Sorbinsäure, Benzoesäure und deren Alkalisalze.

Ein erfindungsgemäßes Getränk kann zudem natürliche und synthetische Antioxidantien beinhalten. Geeignete natürliche Antioxidantien sind beispielsweise Tocopherole, L- Ascorbinsäure, ihre Fettsäureester, wie L-Ascorbylpalmitat, Gallussäureester und Flavonoide. Geeignete synthetische Antioxidantien sind beispielsweise tert.-Butylhydroxyanisol und tert.-Butylhydrochinon.

Besonders bevorzugte der Ernährung oder dem Genuss dienende erfindungsgemäße Zubereitungen (wie oben beschrieben) sind außerdem Suppen (Instantsuppen wie Zwiebel-, Lauch-, Spargel-, Erbsen-, Karotten-, Tomaten-, Hühner-, Rindfleisch-, Champignon-, Fisch-, Krabben- und Krebssuppen, genauso wie Konservensuppen der genannten Geschmacksrichtungen), Soßen (Instantsoßen der Geschmacksrichtungen Rind, Schwein, Lamm, Fisch, Krebs, Krabben, Champignon, Spargel, Zwiebel und Lauch), Würzungen (Bouillon der Geschmacksrichtungen Rind, Schwein, Huhn, Lamm und Gemüse, Aufstreuwürzmischungen verschiedener Arten bzw. Würzungen von Speisesalz), Snacks bzw. Knabberartikel (gebackene oder frittierte Kartoffelchips oder Kartoffelteigprodukte, Extrudate auf Mais- oder Erdnussbasis), Produkte auf Fett- oder Ölbasis (Mayonnaise, Remoulade und Dressings) und Fertiggerichte.

Erfindungsgemäße Zubereitungen können insbesondere Aufstreuwürzungen sein oder darin enthalten sein. Geeignete Aufstreuwürzungen enthalten z.B. synthetische, natürliche oder naturidentische Aromastoffe sowie Trägerstoffe wie z.B. Maltodextrin, Salze wie z.B. Kochsalz, Gewürze wie z.B. Paprika und Pfeffer, Zuckerstoffe oder Zuckeraustauschstoffe bzw. Süßstoffe wie z.B. Saccharin und Geschmacksverstärker wie z.B. Mononatriumglutamat und/oder Inosinmonophosphat.

Die erfindungsgemäßen Zubereitungen (wie oben beschrieben) können hergestellt werden, indem die erfindungsgemäßen bzw. erfindungsgemäß zu verwendenden Verbindungen der Formel (A) als Lösung oder in Form eines Gemisches mit einem festen oder flüssigen Trägerstoff in die der Ernährung, der Mundhygiene oder dem Genuss dienende Zubereitung eingearbeitet werden. Vorteilhafterweise können die somit gegebenenfalls als Lösung vorliegenden erfindungsgemäßen Zubereitungen durch Sprühtrocknung in eine feste Zubereitung überführt werden.

Zur Herstellung einer erfindungsgemäßen Zubereitung (wie oben beschrieben) können gemäß einer bevorzugten Ausgestaltung die erfindungsgemäß zu verwendenden Verbindungen der Formel (A) bzw. vorzugsweise eine erfindungsgemäße Aromastoffkompositionen (wie oben beschrieben) sowie gegebenenfalls andere Bestandteile der erfindungsgemäßen Zubereitung auch zuvor (d.h. vor der Einarbeitung in die Zubereitung) in Emulsionen, in Liposomen, z.B. ausgehend von Phosphatidylcholin, in Microsphären, in Nanosphären oder auch in Kapseln aus einer für Lebens- und Genussmittel geeigneten Matrix, z.B. aus Stärke, Stärkederivaten, anderen Polysacchariden, natürlichen Fetten, natürlichen Wachsen oder aus Proteinen, z.B. Gelatine, eingearbeitet werden. Eine weitere bevorzugte Ausgestaltung kennzeichnet sich dadurch, dass die erfindungsgemäßen bzw. erfindungsgemäß zu verwendenden Verbindungen der Formel (A), insbesondere eine erfindungsgemäße Aromakomposition (wie oben beschrieben), zuvor mit geeigneten Komplexbildnern, beispielsweise mit Cyclodextrinen oder Cyclodextrinderivaten, bevorzugt Beta-Cyclodextrin, komplexiert werden und in dieser Form eingesetzt werden.

Als andere Bestandteile für die erfindungsgemäßen, der Ernährung oder dem Genuss dienenden Zubereitungen können weitere übliche Grund-, Hilfs- und Zusatzstoffe für Nahrungs- oder Genussmittel verwendet werden, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Ei, Knochen, Knorpel, Fisch, Krusten- und Schalentiere, Gemüse, Früchte, Kräuter, Nüsse, Gemüse-oder Fruchtsäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose), Zuckeralkohole (z.B. Sorbit, Mannitol, Xylitol), natürliche oder gehärtete Fette (z.B. Talg, Schmalz, Palmfett, Kokosfett, gehärtetes Pflanzenfett), fette Öle (z.B. Sonnenblumenöl, Erdnussöl, Maiskeimöl, Distelöl, Olivenöl, Walnussöl, Fischöl, Sojaöl, Sesamöl), Fettsäuren oder deren Salze (z.B. Kaliumstearat, Kaliumpalmitat), proteinogene oder nicht-proteinogene Aminosäuren und verwandte Verbindungen (z.B. Taurin, Kreatin, Kreatinin), Peptide, native oder prozessierte Proteine (z.B. Gelatine), Enzyme (z.B. Peptidasen, Glucosidasen, Lipasen), Nukleinsäuren, Nucleotide (Inositolphosphat), geschmacksmodulierende Stoffe (z.B. Natriumglutamat, 2-Phenoxypropionsäure), Emulgatoren (z.B. Lecithine, Diacylglycerole), Stabilisatoren (z.B. Carageenan, Alginat, Johannisbrotkernmehl, Guarkernmehl), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), Bitterstoffe (z.B. Chinin, Coffein, Limonin), Süßstoffe (z.B. Saccharin, Cyclamat, Aspartam, Neotam, Neohesperidindihydrochalkon), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, sowie Riechstoffe, synthetische, natürliche oder naturidentische Aroma- und Geschmackstoffe.

Gemäß einem weiteren Aspekt der vorliegenden Erfindung werden die erfindungsgemäßen Zubereitungen (wie oben beschrieben) vorzugsweise als Halbfertigwaren zur Aromatisierung von (weiteren) Zubereitungen (zum Beispiel zur Herstellung von Fertigwaren) eingesetzt.

Zahnpflegemittel (als Basis für der Mundpflege dienende erfindungsgemäße Zubereitungen wie oben beschrieben) umfassen im Allgemeinen ein abrasives System (Schleif- oder Poliermittel), wie z.B. Kieselsäuren, Calciumcarbonate, Calciumphosphate, Alumiuniumoxide und/oder Hydroxylapatite, oberflächenaktive Substanzen wie z.B. Natriumlaurylsulfat, Natriumlaurylsarcosinat und/oder Cocamidopropylbetain, Feuchthaltemitteln wie z.B. Glycerin und/oder Sorbit, Verdickungsmittel, wie z.B. Carboxymethylcellulose, Polyethylenglycole, Carrageenan und/oder Laponite^{®}, Süßstoffe, wie z.B. Saccharin, Geschmackskorrigenzien für unangenehme Geschmackseindrücke, Geschmackskorrigenzien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Kühlwirkstoffen wie z.B. Menthol, Mentholderivate (z.B. L-Menthol, L-Menthyllactat, L-Menthylalkylcarbonate, Menthonketale, Menthancarbonsäureamide), 2,2,2-Trialkylessigsäureamiden (z.B. 2,2-Diisopropylpropionsäuremethylamid), Icilin und Icilin-Derivate, Stabilisatoren und aktive Wirkstoffe, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen und/oder Natriumbicarbonat oder Geruchskorrigentien.

Kaugummis, als bevorzugte Ausgestaltung einer der Mundpflege dienenden erfindungsgemäßen Zubereitung (wie oben beschrieben), umfassen im Allgemeinen eine Kaugummibase, d.h. eine beim Kauen plastisch werdende Kaumasse, Zucker verschiedener Arten, Zuckeraustauschstoffe, andere süß schmeckende Stoffe, Zuckeralkoholen, Geschmackskorrigenzien für unangenehme Geschmackseindrücke, andere Geschmacksmodulatoren für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Feuchthaltemittel, Verdicker, Emulgatoren, Aromen und Stabilisatoren oder Geruchskorrigentien.

Die erfindungsgemäßen bzw. die erfindungsgemäß zu verwendenden Verbindungen der Formel (A) können jeweils als Reinstoffe oder in Form von Lösungen vorliegen. Als Lösungsmittel werden dabei bevorzugt Wasser, Methanol, Ethanol, Propylenglykol, Glycerin, Aceton, Dichlormethan, Diethylether, Hexan, Heptan, Triacetin, pflanzliche Öl oder Fette wie beispielsweise Pflanzentrigycerid (gegebenenfalls kosher), superkritisches Kohlendioxid oder auch Gemische der zuvor genannten Lösungsmittel verwendet. Die ohnehin vorteilhafterweise sehr lange Haltbarkeit der Reinstoffe erfindungsgemäßer bzw. erfindungsgemäß zu verwendender Verbindungen der Formel (A) von 12 Monaten kann in Lösung vorheilhafterweise auf 24 Monate oder mehr erhöht werden.

Ein weiterer Aspekt der vorliegenden Erfindung betrifft ein Verfahren zum Vermitteln, Modifizieren und/oder Verstärken eines Geruchs und/oder Geschmacks (vorzugsweise eines Geruchs und/oder Geschmacks mit einer blumigen und/oder fruchtigen Note), wobei eine Menge
- einer Verbindung der Formel (A)
   oder
- einer erfindungsgemäßen Mischung aus zwei oder mehr Verbindungen der Formel (A),
   wie oben beschrieben,
   oder
- einer erfindungsgemäßen Verbindung der Formel (III) oder (IV) oder einer erfindungsgemäßen Mischung wie oben beschrieben,
   oder
- einer erfindungsgemäßen Riech- oder Aromastoffkomposition wie oben beschrieben mit einem Erzeugnis in Kontakt gebracht oder gemischt wird.

Besonders bevorzugt ist ein erfindungsgemäßes Verfahren (wie oben beschrieben) zum Vermitteln, Modifizieren und/oder Verstärken einer, zwei oder sämtlicher der Geruchs- und/oder Geschmacksnoten Grapefruit, tropische Früchte und schwarze Johannisbeere.

Für die bevorzugt einzusetzenden Verbindungen der Formel (A) bzw. Mischungen bzw. Riech- und Aromastoffkompositionen gilt jeweils das vorangehend Gesagte entsprechend.

Die vorliegende Erfindung betrifft auch ein Verfahren zum Modifizieren und/oder Verstärken eines Geruchs und/oder Geschmacks mit einer blumigen und/oder fruchtigen Note, wobei ein oder mehrere Riech- oder Aromastoff(e) mit blumiger und/oder fruchtiger Note mit einer Menge an Verbindungen der Formel (A) vermischt wird bzw. werden, die ausreicht, den Geruchs- und/oder Geschmackseindruck des bzw. der Riech- bzw. Aromastoffe sensorisch zu verstärken.

Im Folgenden wird die vorliegende Erfindung an Hand von Beispielen näher erläutert, wobei diese die Erfindung in keiner Weise einschränken.

### Beispiele:

Sofern nicht anders angegeben beziehen sich sämtliche der folgenden Angaben auf das Gewicht.

Verwendete Abkürzungen: DPG = Dipropylenglycol; IPM = Isopropylmyristat; TEC = Triethylcitrat.

Die im Folgenden als erfindungsgemäße "Mischung **X"** bezeichnete Zusammensetzung enthielt nach eigenen Untersuchungen 57,5 Gew.-% an Verbindung (Id), 33,9 Gew.-% an Verbindung (Ib), 3,2 Gew.-% an Verbindung (IIId) und 2,3 Gew.-% an Verbindung (IIIb). Eine solche Mischung ist eine besonders bevorzugte erfindungsgemäße bzw. erfindungsgemäß zu verwendende Mischung. Eine erfindungsgemäße Mischung **X** kann in Analogie zu Beispiel 1 erhalten werden, wobei die Säulenchromatographie in der Weise durchgeführt wird, dass nicht nur eine Verbindung der Formel (I), sondern auch eine Verbindung der Formel (III) in die Produktfraktion aufgenommen wird.

### Beispiel 1: Synthese von 1-p-Menthen-3-thiol (Verbindung der Formel (I))

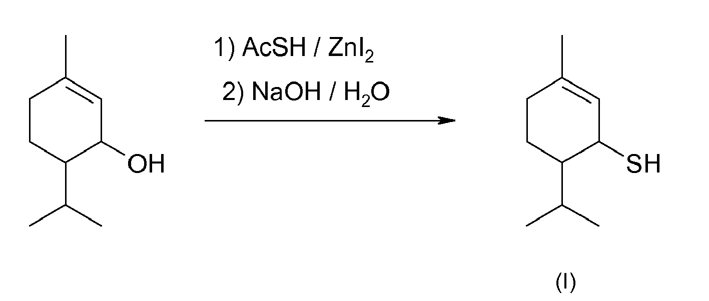

Zu einer Lösung von 19,2 g (0,124 mol) Piperitol in 250 ml wasserfreiem Dichlormethan wurde unter Rühren 11,7 g (0,15 mol) Thioessigsäure und 20,2 g (0,06 mol) wasserfreies Zinkiodid gegeben. Das erhaltene Reaktionsgemisch wurde 5 h bei Raumtemperatur (ca. 20°C) gerührt, mit 250 ml dest. Wasser versetzt, die organische Phase wurde abgetrennt und die wässrige Phase noch zweimal mit je 50 ml Dichlormethan extrahiert.

Die vereinigten organischen Phasen wurden mit gesättigter wässriger Natriumhydrogencarbonat-Lösung und anschließend gesättigter wässriger Kochsalzlösung gewaschen, über Natriumsulfat getrocknet und im Vakuum eingeengt. Zu einer gerührten Lösung des verbliebenen Rückstandes (26,5 g) in 200 ml Aceton wurden bei Raumtemperatur 200 ml 3-molare wässrige Natronlauge getropft, und das erhaltene Reaktionsgemisch wurde anschließend 4 h bei Raumtemperatur gerührt, mit 3-molarer wässriger Salzsäure neutralisiert und dreimal mit je 50 ml Dichlormethan extrahiert. Die (anschließend vereinigten) Extrakte wurden über Natriumsulfat getrocknet und im Vakuum eingeengt. Durch Säulenchromatographie (Kieselgel, Cyclohexan / Ethylacetat 3:1) wurde aus dem verbliebenen Rückstand eine Probe mit Verbindungen der Formel (I) (2,72 g; Reinheit nach GC: 99,4%) erhalten.

### Beispiel 2: Synthese von 1-p-Menthen-4-thiol (Verbindung der Formel (II))

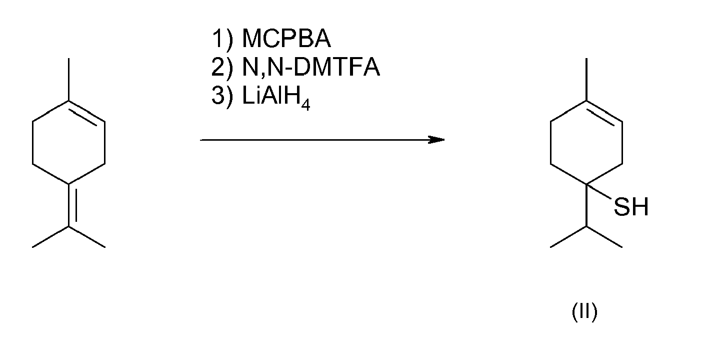

Zu einer Lösung von 6,8 g (0,05 mol) Terpinolen in 100 ml wasserfreiem Dichlormethan wurde unter Rühren bei 0°C eine Lösung von 11,8 g (0,05 mol) m-Chlorperbenzoesäure (77%ig) MCPBA in 150 ml wasserfreiem Dichlormethan getropft und 30 Minuten bei dieser Temperatur sowie weitere 30 Minuten bei Raumtemperatur gerührt. Das erhaltene Reaktionsgemisch wurde 30 Minuten bei Raumtemperatur mit 100 ml 10%iger wässriger Natriumcarbonatlösung gerührt, die organische wurde Phase abgetrennt, zweimal mit je 50 ml Wasser gewaschen, über Natriumsulfat getrocknet und im Vakuum eingeengt. Der Rückstand (12 g, nach GC 82% Produkt enthaltend) wurde in 50 ml wasserfreiem Dichlormethan aufgenommen und mit 11,6 g N,N-Dimethylthioformamid (N,N-DMTFA) versetzt. Unter Rühren und Stickstoff-Schutzgasatmosphäre wurden 0,74 g Trifluoressigsäure zugetropft. Das Reaktionsgemisch wurde anschließend 3 h bei 60°C unter Stickstoff gerührt. Nach dem Abkühlen auf Raumtemperatur wurde das Lösungsmittel im Vakuum entfernt, der verbliebene Rückstand in 200 ml n-Hexan aufgenommen und die erhaltene Lösung mit 300 ml Wasser gewaschen, über Natriumsulfat getrocknet und im Vakuum eingeengt. Eine Lösung von 3,0 g des erhaltenen Rückstandes (Gesamtmenge: 8,2 g) in 5 ml Tetrahydrofuran wurde unter Stickstoff-Schutzgasatmosphäre zu einer siedenden Suspension von 0,4 g Lithiumaluminiumhydrid (LiAlH₄) in Tetrahydrofuran getropft. Das erhaltene Reaktionsgemisch wurde anschließend 5 h unter Rückfluss erhitzt, nach Abkühlen auf Raumtemperatur mit Wasser hydrolysiert und im Vakuum eingeengt. Der verbliebene wasserhaltige Rückstand wurde dreimal mit je 20 ml Ether extrahiert. Die (anschließend vereinigten) Ether-Extrakte wurden mit gesättigter wässriger Natriumhydrogencarbonatlösung gewaschen, über Natriumsulfat getrocknet und im Vakuum eingeengt. Durch Säulenchromatographie des hierbei erhaltenen Rückstandes (Kieselgel, Cyclohexan / Ethylacetat 4:1) wurde eine Probe mit Verbindungen der Formel (II) (1,36 g; Reinheit nach GC: 99,1 %) erhalten.

### Beispiel 3: Synthese von 1-p-Menthen-5-thiol (Verbindung der Formel (III))

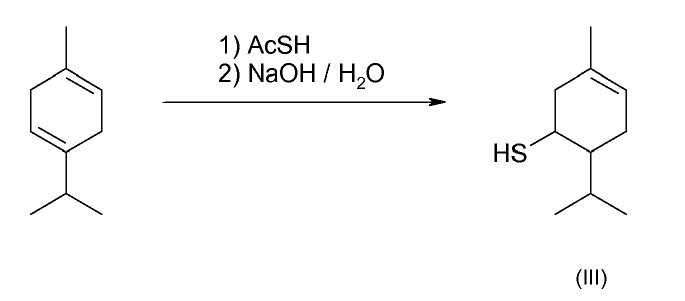

Zu 29,2 g (0,21 mol) gamma-Terpinen wurden unter Rühren bei 0°C 32,1 g (0,41 mol) Thioessigsäure getropft. Das erhaltene Gemisch wurde anschließend 5 Tage bei Raumtemperatur gerührt, in 200 ml Aceton gelöst, mit 200 ml 3-molarer wässriger Natronlauge versetzt, 5 h bei Raumtemperatur gerührt und mit 3-molarer wässriger Salzsäure neutralisiert. Die organische Phase wurde anschließend abgetrennt. Die wässrige Phase wurde zweimal mit je 50 ml Dichlormethan extrahiert und die vereinigten organischen Phasen wurden über Natriumsulfat getrocknet und im Vakuum eingeengt. Durch Säulenchromatographie (Kieselgel, Cyclohexan / Ethylacetat 8:1) des hierbei erhaltenen Rückstandes wurde eine Probe mit Verbindungen der Formel (III) (0,39 g; Reinheit nach GC: 99,8%) erhalten.

### Beispiel 4: Synthese von 1-p-Menthen-6-thiol (Verbindung der Formel (IV))

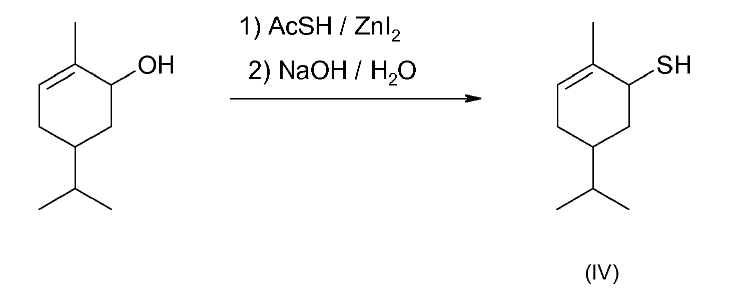

Zu einer Lösung von 19,2 g (0,124 mol) 8,9-Dihydrocarveol in 250 ml wasserfreiem Dichlormethan wurden unter Rühren 11,7 g (0,15 mol) Thioessigsäure und 20,2 g (0,06 mol) wasserfreies Zinkiodid zugegeben. Das erhaltene Reaktionsgemisch wurde 5 h bei Raumtemperatur gerührt und anschließend mit 250 ml dest. Wasser versetzt. Die organische Phase wurde abgetrennt und die wässrige Phase noch zweimal mit je 50 ml Dichlormethan extrahiert.

Die vereinigten organischen Phasen wurden mit gesättigter wässriger Natriumhydrogencarbonat-Lösung und anschließend mit gesättigter wässriger Kochsalzlösung gewaschen, über Natriumsulfat getrocknet und im Vakuum eingeengt. Zu einer gerührten Lösung des verbliebenen Rückstandes (19,7 g) in 200 ml Aceton wurden bei Raumtemperatur 200 ml 3-molare wässrige Natronlauge getropft. Das erhaltene Reaktionsgemisch wurde anschließend 4 h bei Raumtemperatur gerührt, mit 3-molarer wässriger Salzsäure neutralisiert und dreimal mit je 50 ml Dichlormethan extrahiert. Die vereinigten organischen Extrakte wurden über Natriumsulfat getrocknet und im Vakuum eingeengt. Durch Säulenchromatographie des so erhaltenen Rückstandes (Kieselgel, Cyclohexan / Ethylacetat 3:1) wurde eine Probe mit Verbindungen der Formel (IV) (1,73 g; Reinheit nach GC: 99,6%) erhalten.

### Anwendungsbeispiele:

**Parfümöl D: Orientalischer Damenduft D / Parfümöl P: Duft "Pink Grapefruit":**

| **Bestandteil / Riechstoff(e)** | **Parfümöl D** (erfindungsgemäß) | **Parfümöl P** (erfindungsgemäß) |
|---|---|---|
| Agrunitril | | 30,0 |
| Aldehyd C8 (n-Octanal) | | 4,0 |
| Aldehyd C12 (n-Dodecanal) | | 2,0 |
| Aldehyd C14 sogenannt, 10% in TEC | 2,0 | 8,0 |
| Allylamylglycolat, 10% in TEC | 20,0 | 15,0 |
| Allylcapronat | | 15,0 |
| Allylcyclohexylpropionat | | 15,0 |
| Abrein, 10% in DPG | 10,0 | |
| Benzylacetat | | 2,0 |
| Bergamottöl | 25,0 | |
| Cashmeran^{®}, 10% in DPG | 6,0 | |
| Cedernholzöl | 3,0 | |
| Citral, 10% in DPG | 2,0 | |
| Citronellol | | 3,0 |
| Citronenöl, italienisch | 2,0 | |
| Citrylal | | 25,0 |
| Claritone^{®} | | 35,0 |
| Cumarin | 12,0 | |
| Cyclamenaldehyd | 2,0 | |
| p-Cymol | | 5,0 |
| Damascenon, 10% in DPG | 2,0 | |
| Beta-Damascon, 10% in DPG | 5,0 | |
| Davanaöl, 10% in DPG | 5,0 | |
| Gamma-Decalacton, 10% in DPG | 4,0 | |
| Trans-2-Decenal, 1% in DPG | | 5,0 |
| Dihydromyrceol | | 25,0 |
| Dipropylenglycol (DPG) | ad 1000 | |
| Trans-2-Dodecenal, 10% in TEC | | 95,0 |
| Ebanol^{®}, 10% in DPG | 4,0 | |
| Ethylbutyrat, 10% in DPG | | 2,0 |
| Ethylmaltol | 6,0 | |
| Ethylvanillin, 10% in DPG | 12,0 | 1,0 |
| Exaltenon, 10% in DPG | 2,0 | |
| Galaxolid^{®}, 50% in DEP | 38,0 | |
| Geraniumöl | 4,0 | |
| Grapefruit Base | 7,0 | 35,0 |
| Methyldihydrojasmonat (Hedion^{®}) | 44,0 | |
| Cis-3-Hexenol, 10% in TEC | | 2,0 |
| Cis-3-Hexenylacetat, 10% in DPG | 2,0 | |
| Cis-3-Hexenylsalicylat | 2,0 | |
| Alpha-Hexylzimtaldehyd | 5,0 | |
| Iso E Super^{®} | 3,0 | |
| Krauseminzöl (Spearmintöl), natürlich, USA, 10% in TEC | | 5,0 |
| Ligustral^{®}, 10% in DPG | 6,0 | |
| Lilial^{®} | 24,0 | |
| Limettenöl | | 10,0 |
| Linalool | 10,0 | |
| Linalylacetat | 12,0 | |
| Lyral | 2,0 | |
| I-Menthol | | 30,0 |
| Muskatnussöl, 10% in DPG | 12,0 | |
| Nootkaton | | 1,0 |
| Orangenöl, brasilianisch | 8,0 | |
| Patchouliöl, indonesisch | 75,0 | |
| Resedafol | | 7,0 |
| Rosenoxid, high-cis | | 4,0 |
| Sandelholzöl | 25,0 | |
| Styrolylacetat | 5,0 | 3,0 |
| Terpineol | | 3,0 |
| Terpinylacetat | 5,0 | |
| Thiocineol, 10% in TEC | | 3,0 |
| 8,3-Thiomenthanon, 10% in TEC | | 5,0 |
| Thymol, 10% in TEC | | 5,0 |
| Tonalid^{®} | 27,0 | |
| Trans-2-Tridecenal | | 1,0 |
| Triethylcitrat (TEC) | | ad 1000 |
| Vanillin | 9,0 | |
| Vetikolacetat | | 25,0 |
| Mischung **X**, 1% in TEC | 1,0 | 0,3 |
| TOTAL: | 1000,0 | 1.000,0 |

In den folgenden Anwendungsbeispielen **A1** bis **A5** wurden die Parfumöle D und P in die entsprechenden Formulierungen eingearbeitet.

**Anwendungsbeispiel A1: Allzweckreiniger**

| **Bestandteil** | **Hersteller** | **Chemischer** **Name** | **Funktion** | **Gew.-** **%** |
|---|---|---|---|---|
| Entionisiertes Wasser | | Water | Lösungsmittel | 59,6 |
| Mergal K9N | Troy Chemie, Seelze | 5-Chloro-2-methyl-3-(2H)-isothiazolone und 2-methyl-3-(2H)-isothiazolone | Konservierungsmittel | 0,1 |
| Trinatriumcitrat Dihydrat | Verschiedene | Tri Sodium Citrate Dihydrate | Komplexbildner | 3,0 |
| Zetesol NL-2 | Zschimmer & Schwarz, Deutschland | Fatty alcohol C12-14-sulfate, Sodium | Anionisches Tensid | 30,0 |
| Imbentin C/125/055 | Dr. W. Kolb AG Chem. | Fatty alcohol C12-C15, 8EO | Nichtionisches Tensid | 5,0 |
| Ethanol 96% | Verschiedene | Ethanol | Lösungsmittel | 2,0 |
| Parfumöl D oder P | Symrise | | Parfum (Fragrance) | 0,3 |

**Anwendungsbeispiel A2: Shampoo**

| **Bestandteil** | **Hersteller** | **INCI-Name** | **Gew.-%** |
|---|---|---|---|
| Entionisiertes Wasser | | Water | 71,5 |
| Plantacare PS 10 | Cognis Deutschland GmbH | Sodium Laureth Sulfate, Lauryl Glucoside | 20,0 |
| Euperlan PK 771 PK | Cognis Deutschland GmbH | Glycol Distearate, Sodium Lauryl Sulfate, Cocamide MEA, Laureth-10 | 6,0 |
| Dragocid Liquid | Symrise | Phenoxyethanol, Methylpa-raben, Ethylparaben, Butyl-paraben, Propylparaben, Isobutylparaben | 0 5 |
| Natriumchlorid | | Sodium Chloride | 1,4 |
| Citronensäure Monohydrat kristallin | | Citric Acid | 0,1 |
| Parfumöl D oder P | Symrise | Parfum (Fragrance) | 0,5 |

**Anwendungsbeispiel A3: Duschgel**

| **Bestandteil** | **Hersteller** | **INCI-Name** | **Gew.-%** |
|---|---|---|---|
| Entionisiertes Wasser | | Wasser | 76,3 |
| Plantacare PS 10 | Cognis Deutschland GmbH | Sodium Laureth Sulfate, Lauryl Glucoside | 20,0 |
| Dragocid Liquid | Symrise | Phenoxyethanol, Methylparaben, Ethylparaben, Butylparaben, Propylparaben, Isobutylparaben | 0,5 |
| Natrium Chlorid | | Sodium Chloride | 1,4 |
| Citronensäure Monohydrat | | Citric Acid | 1,3 |
| kristallin | | | |
| Parfumöl D oder P | Symrise | Parfum (Fragrance) | 0,5 |

**Anwendungsbeispiel A4: Weichspüler**

| **Bestandteil** | **Hersteller** | **Chemischer Name** | **Funktion** | **Gew.-%** |
|---|---|---|---|---|
| Entionisiertes Wasser | | Wasser | Lösungsmittel | 72,4 |
| Rewoquat WE 18 | Evonic Gold-schmidt GmbH | Dialkylesteram-monium-ethosulfate | Kationisches Tensid | 16,6 |
| Mergal K9N | Honeywell Austria GmbH | 5-Chloro-2-methyl-3-(2H)-isothiazolon und 2-methyl-3-(2H)-isothiazolon | Konservierungs-mittel | 0,10 |
| Dow Corning 1520 Antifoam | Dow Corning GmbH, Deutschland | Polydimethyl-siloxane | Entschäumer | 0,30 |
| Magnesium Chlorid 1/oige Lösung | | Magnesiumchlorid-Lösung | Konsistenzgeber | 10,00 |
| Parfumöl D oder P | Symrise | | Parfum (Fragran-ce) | 0,60 |

**Anwendungsbeispiel A5: Hair Conditioner mit UV-Schutz**

| **Komponente** | **INCI Name** | **Gew.-%** |
|---|---|---|
| Lanette O | Cetearyl Alcohol | 4,00 |
| Dragoxat 89 | Ethylhexyl Isononanoate | 4,00 |
| Emulsiphos | Potassium Cetyl Phosphate, Hydrogenated Palm Glyce-rides | 0,50 |
| Natrosol 250 HR | Hydroxyethylcellulose | 0,25 |
| Neo Hehopan^{®} Hydro | Phenylbenzimidazole Sulfonic Acid | 2,00 |
| L-Arginin | Arginine | 1,20 |
| Benzophenon-4 | Benzophenone-4 | 0,50 |
| Neo Heliopan^{®} AP | Disodium Phenyl Dibenzimidazole Tetrasulfonate | 0,50 |
| Edeta BD | Disodium EDTA | 0,05 |
| Dragocide Liquid | Phenoxyethanol (and) Methylparaben (and) Butyparaben (and) Ethyparaben (and) Propylparaben | 0,80 |
| Dow Corning 949 Cationic Emulsion | Amodimethicone, Cetrimonium Chloride, Trideceth-12 | 2,00 |
| Dow Corning 5200 | Laurylmethicone Copolyol | 0,50 |
| Parfumöl D oder P | Parfum | 0,95 |
| Wasser | Water (Aqua) | Ad 100 |

**Anwendungsbeispiel A6: Kaugummis**

| **Komponente** | Gew.-% | |
|---|---|---|
| | **A6a** | **A6b** |
| Kaugummibase | 22,0 | 30,0 |
| Glycerin | 0,5 | 1,0 |
| Aroma aus natürlichem Pfefferminzöl und natürlichem Spearmintöl (enthaltend I-Menthol, Menthylacetat, Menthon sowie I-Carvon) | 0,7 | 0,2 |
| Sprühgetrocknetes I-Menthol (enthaltend 20% I-Menthol) | 0,25 | |
| Glucosesirup | 16,5 | |
| Puderzucker | ad 100 | |
| Grapefruitaroma enthaltend je 1250 ppm an Verbindung der Formel (I) und Verbindung der Formel (III) | 0,55 | - |
| Aroma von Passionsfrucht und schwarzer Johannisbeere mit 2400 ppm an Verbindung der Formel (III) | - | 1,10 |
| Sorbitol-Pulver | | ad 100 |
| Palatinit | | 9,5 |
| Xylitol | | 2,0 |
| Mannitol | | 3,0 |
| Aspartam | | 0,1 |
| Acesulfam K | | 0,1 |
| Emulgum / Emulgator | | 0,3 |
| Sorbitol 70%, in Wasser | | 14,0 |

### Anwendungsbeispiele A7 bis A11: Aromatisierte Zubereitungen

**A7** = Instant-Getränkepulver
**A8** = Zuckerfreies Instant-Getränkepulver
**A9** = Kohlensäurehaltiges Getränk
**A10** = Soja-Frucht - Getränk
**A11** = Fettarmer Joghurt

| **Komponente** | Gew.-% | | | | |
|---|---|---|---|---|---|
| | **A7** | **A8** | **A9*** | **A10** | **A11** |
| Verbindung der Formel (III) | | 70 ppm | | | |
| Verbindung der Formel (I) | | | | | 6 ppm |
| Mischung **X** | 45 ppm | | 5 ppm | 8 ppm | |
| Zucker (Saccharose) | ad 100 | | 8,00 | 5,00 | 5,00 |
| Zitronensäure | 4,00 | 33,33 | 0,20 | | |
| Trinatriumcitrat | 0,26 | | | | |
| Tricalciumphosphat | 0,22 | | | | |
| Ascorbinsäure (Vitamin C) | 0,24 | 0,44 | | | |
| Trübungsmittel und Tita-niumdioxid (E 171) | 0,20 | | | | |
| Xanthan gum (E 415) | 0,072 | | | | |
| Natrium-Carboxymethyl cellulose (E 467) | 0,064 | | | | |
| Pektin (E 440) | 0,04 | | | | |
| Sprühgetrocknetes Grapefruit-Aroma mit gelbem Farbstoff Tartrazin | 0,40 | | | | |
| Sprühgetrocknetes Johan-nisbeer-Aroma mit rotem Farbstoff | | 11,50 | | | |
| Zitronen und Limetten- Aroma | | | 0,01 | | |
| D-Limonen | | | 0,005 | | |
| Maltodextrin (Pulver) | | ad 100 | | | |
| Aspartam | | 3,30 | | | |
| Hesperetin (1% in 1,2-Propylenglycol) | | | 0,05 | | |
| Phloretin (1% in 1,2-Propylenglycol) | | | 0,05 | | |
| Ethylhydroxymethylfuranon | | | 0,01 ppb | | |
| Vanille-Aroma | | | | 0,10 | 0,125 |
| Vanillin | | | 15 ppb | | |
| Maltol | | | 350 ppb | | |
| 2,5-Dimethyl-4-hydroxy-2H-furan-3-on | | | 3 ppb | | |
| 1,2-Propylenglycol | | | 0,10 | | |
| Mischung von Fruchtsaft-Konzentraten | | | | 45,00 | |
| Soja Pulver | | | | 5,00 | |
| Joghurt (1,5% Fett) | | | | | ad 100 |
| Wasser | | | ad 100 | ad 100 | |

| | | | | | |
|---|---|---|---|---|---|
| * Carbonisierung / Versetzen mit Kohlensäure nach Abfüllung in Flaschen. | | | | | |

## Patentansprüche

1. Verwendung
- einer Verbindung der Formel (A) 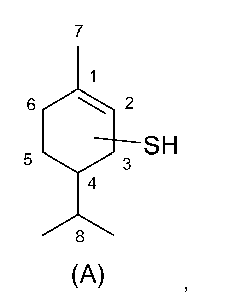 wobei die Thiolgruppe der Verbindung der Formel (A) mit dem Kohlenstoffatom in Position 3, 4, 5 oder 6 verknüpft ist,
oder
- einer Mischung aus zwei oder mehr Verbindungen der Formel (A), wobei die Thiolgruppe jeder Verbindung der Formel (A) jeweils unabhängig voneinander mit dem Kohlenstoffatom in Position 3, 4, 5 oder 6 verknüpft ist,
als Riech- und/oder Aromastoff zum Vermitteln, Modifizieren und/oder Verstärken einer, zwei oder sämtlicher der Geruchs- und/oder Geschmacksnoten Grapefruit, tropische Früchte und schwarze Johannisbeere.

2. Verwendung nach Anspruch 1, wobei die Verbindung der Formel (A) bzw. eine, mehrere oder sämtliche Verbindungen der Formel (A) der Mischung ausgewählt ist bzw. jeweils unabhängig voneinander ausgewählt sind aus der Gruppe bestehend aus Verbindungen der Formeln (I) und (III)

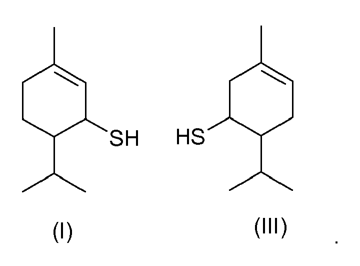

3. Verwendung nach Anspruch 1 oder 2 wobei die Verbindung der Formel (A) bzw. eine, mehrere oder sämtliche Verbindungen der Formel (A) der Mischung ausgewählt ist bzw. jeweils unabhängig voneinander ausgewählt sind aus der Gruppe bestehend aus den Verbindungen der Formeln (Ia), (Ib), (Ic), (Id), (IIIa), (IIIb), (IIIc) und (IIId)

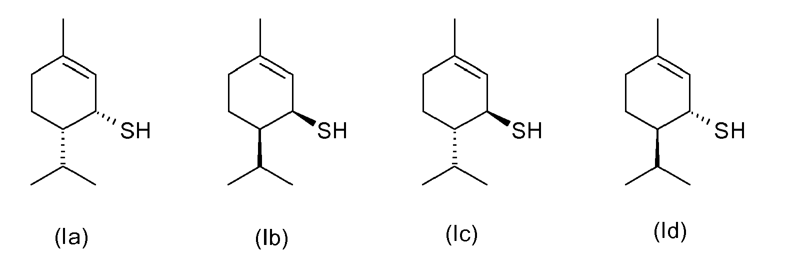

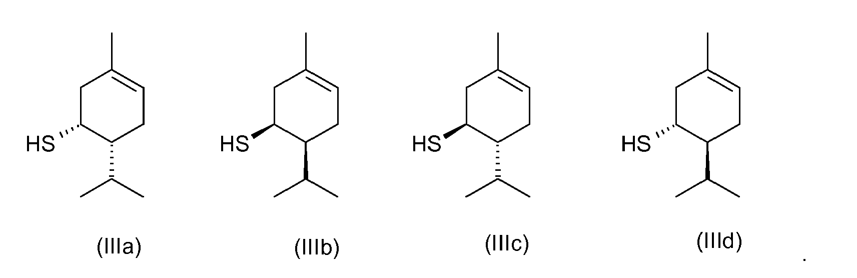

4. Verwendung einer Mischung aus zwei oder mehr Verbindungen der Formel (A) nach einem der vorangehenden Ansprüche, wobei die Mischung
eine oder mehrere Verbindungen der Formel (A), ausgewählt aus der Gruppe bestehend aus den Verbindungen der Formeln (Ia), (Ib), (Ic) und (Id), und/oder eine oder mehrere Verbindungen der Formel (A), ausgewählt aus der Gruppe bestehend aus den Verbindungen der Formeln (IIIa), (IIIb), (IIIc) und (IIId),
vorzugsweise ausgewählt aus der Gruppe bestehend aus den Verbindungen der Formeln (Ib), (Id), (IIIb) und (IIId),
umfasst oder daraus besteht.

5. Verwendung nach Anspruch 4, wobei die Mischung eine oder mehrere Verbindungen der Formel (A), ausgewählt aus der Gruppe bestehend aus den Verbindungen der Formeln (la), (Ib), (Ic) und (Id), und eine oder mehrere Verbindungen der Formel (A), ausgewählt aus der Gruppe bestehend aus den Verbindungen der Formeln (IIIa), (IIIb), (IIIc) und (IIId) umfasst oder daraus besteht und
wobei das Gewichtsverhältnis der Gesamtmenge an Verbindungen der Formel (III) zur Gesamtmenge an Verbindungen der Formel (I) in der Mischung vorzugsweise im Bereich von 1 : 99 bis 20 : 80 liegt, vorzugsweise im Bereich von 2 : 98 bis 10 : 90, besonders bevorzugt im Bereich von 3 : 97 bis 8 : 92.

6. Verwendung nach einem der vorangehenden Ansprüche in einer Riech- und/oder Aromastoffkomposition oder einer der Ernährung oder dem Genuss dienenden Zubereitung.

7. Verbindung der Formel (III) oder (IV) 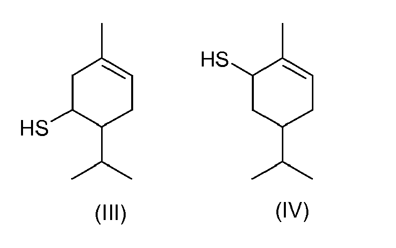 oder Mischung umfassend oder bestehend aus einer oder mehreren Verbindungen ausgewählt aus der Gruppe bestehend aus Verbindungen der Formeln (III) und (IV).

8. Mischung nach Anspruch 7, ferner umfassend eine oder mehrere Verbindungen ausgewählt aus der Gruppe bestehend aus Verbindungen der Formeln (I) und (II)

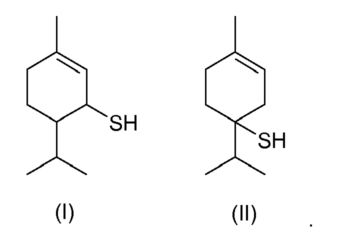

9. Riech- oder Aromastoffkomposition, bestehend aus oder umfassend
(i) eine Verbindung der Formel (A) oder eine Mischung aus zwei oder mehr Verbindungen der Formel (A), wie jeweils in einem der Ansprüche 1 bis 8 definiert, sowie
(ii) einen, zwei, drei oder mehr weitere Riech- und/oder Aromastoffe, wobei der weitere bzw. die weiteren Riech- und/oder Aromastoff(e) keine Verbindung(en) der Formel (A) ist bzw. sind,
mit der Maßgabe, dass die Riech- bzw. Aromastoffkomposition weder p-Menthenthiol-1,8 noch 1,8-*epi*-thio-*p*-Methan umfasst, und mit der weiteren Maßgabe, dass die Riech- oder Aromastoffkomposition bei Anwesenheit von Hexan und/oder Ethylacetat einen, zwei, drei oder mehr weitere Riech- und/oder Aromastoffe umfasst, wobei der weitere bzw. die weiteren Riech- und/oder Aromastoff(e) keine Verbindung(en) der Formel (A) ist bzw. sind.

10. Riech- oder Aromastoffkomposition nach Anspruch 9, wobei die Gesamtmenge an Verbindungen der Formel (A) ausreicht, um in der Riech- bzw. Aromastoffkomposition den sensorischen Eindruck des weiteren bzw. eines, mehrerer oder sämtlicher weiteren Riech- und/oder Aromastoffe zu modifizieren und/oder zu verstärken.

11. Riech- oder Aromastoffkomposition nach Anspruch 9 oder 10, wobei
- der weitere bzw. einer, mehrere oder sämtliche weiteren Riechstoff(e) ausgewählt ist bzw. sind aus der Gruppe bestehend aus blumigen und fruchtigen Riechstoffen und/oder
- der weitere bzw. einer, mehrere oder sämtliche weiteren Aromastoff(e) ausgewählt ist bzw. sind aus der Gruppe bestehend aus blumigen und fruchtigen Aromastoffen.

12. Der Ernährung, dem Genuss oder der Mundpflege dienende Zubereitung, umfassend eine sensorisch wirksame Menge an
- einer Verbindung der Formel (A)
oder
- einer Mischung aus zwei oder mehr Verbindungen der Formel (A),
wie in einem der Ansprüche 1 bis 6 definiert,
oder
- einer Verbindung der Formel (III) oder (IV)
oder einer Mischung
nach einem der Ansprüche 7 oder 8,
oder
- einer Riech- oder Aromastoffkomposition nach einem der Ansprüche 9 bis 11 sowie
- einen oder mehrere weitere zum Verzehr geeignete Bestandteile.

13. Parfümierter Artikel, umfassend eine Riech- oder Aromastoffkomposition nach einem der Ansprüche 9 bis 11, wobei der Artikel ausgewählt ist aus der Gruppe bestehend aus Eau de Parfums, Eau de Toilettes, Rasierwässer, Eau de Colognes, Pre-shave-Produkte, Splash-Colognes, saure, alkalische und neutrale Reinigungsmittel, Geschirrspülmittel, Bad- und Sanitärreiniger, feste und flüssige WC-Reiniger, flüssige Waschmittel, pulverförmige Waschmittel, Wäscheweichspüler, Waschseifen, Waschtabletten, Oberflächendesinfektionsmittel, Luftverbesserer, Aerosolspray, Körperpflegemittel, Duschgele, Shampoos, Rasierseifen, Rasierschäume, Badeöle, kosmetische Emulsionen, Hautcremes- und -lotionen, Gesichtscremes und -lotionen, Sonnenschutzcremes- und -lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarspülungen, permanente und semipermanente Haarfärbemittel, Haarverformungsmittel, Haarwässer, Achselsprays, Roll-ons, Deosticks, Deocremes, Make-ups, Lippenstifte, Mascara, Kerzen oder Lampenöle..

14. Verfahren zum Vermitteln, Modifizieren und/oder Verstärken eines Geruchs und/oder Geschmacks, wobei eine Menge
- einer Verbindung der Formel (A)
oder
- einer Mischung aus zwei oder mehr Verbindungen der Formel (A),
wie in einem der Ansprüche 1 bis 6 definiert,
oder
- einer Verbindung der Formel (III) oder (IV)
oder einer Mischung
nach einem der Ansprüche 7 oder 8,
oder
- einer Riech- oder Aromastoffkomposition nach einem der Ansprüche 9 bis 11 mit einem Erzeugnis in Kontakt gebracht oder gemischt wird.

15. Verfahren nach Anspruch 14 zum Vermitteln, Modifizieren und/oder Verstärken einer, zwei oder sämtlicher der Geruchs- und/oder Geschmacksnoten Grapefruit, tropische Früchte und schwarze Johannisbeere.
